# EUROPEAN PATENT APPLICATION

(11) **EP 3 410 117 A1**
(43) Date of publication of application: **05.12.2018**
(21) Application number: 17305648.2
(22) Date of filing: 02.06.2017
(51) Int. Cl.: G01N 33/569

(54) **DETECTION OF BORRELIA BURGDORFERI SENSU LATO IN PATIENTS WITH LATE DISSEMINATED LYME INFECTION**

(71) Applicant: Université de Strasbourg, 67000 Strasbourg (FR); CENTRE NATIONAL DE LA RECHERCHE SCIENTIFIQUE, 75016 Paris (FR)
(72) Inventor: BOULANGER, Nathalie, 67140 Heiligenstein (FR); SABATIER, Laurence, 67120 Molsheim (FR); WESTERMANN, Benoît, 57150 Creutzwald (FR)
(74) Representative: Novagraaf Technologies

(57) **Abstract**

The presence invention relates to an *ex vivo* method of detecting a *Borrelia burgdorferi* sensu lato active infection in a skin sample of a subject, said skin sample being treated with an immunosuppressive dermocorticoid before sampling, comprising the step of detecting, in said skin sample, at least one protein of *Borrelia burgdorferi* sensu lato chosen in the group comprising VIsE, flagellin, GAPDH, flagellar filament outer layer protein, L-lactate dehydrogenase, enolase, oligopeptide ABC transporter periplasmic oligopeptide-binding protein, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog thereof, and DbpA.
The invention also relates to a set of polypeptides of *Borrelia burgdorferi* sensu lato and to the use of the set of polyeptides in an *ex vivo* method of detecting *Borrelia burgdorferi* sensu lato in a subject likely to present an active Lyme infection.

## Description

### Technical field

The present invention refers to an *ex vivo* method of detecting a *Borrelia burgdorferi* sensu lato active infection in a skin sample of a subject.

Therefore, the present invention has utility in medical field.

In the description below, the references into brackets ([ ]) refer to the listing of references situated at the end of the text.

### Background of the invention

Lyme borreliosis is the first arthropod-borne disease of the Northern Hemisphere. The bacteria, *Borrelia burgdorferi* sensu lato (sl), responsible of the disease, are transmitted by hematophagous bite of a hard tick *Ixodes* spp. (Radolf et al. 2012 **([1])**. Efficient interactions between pathogen, vector and host are essential for the persistence of the bacteria in Nature. The blood meal induces a rapid multiplication of the bacteria in the midgut of infected tick (Piesman et al. 2001 **([2])**). Then, *Borrelia* migrates to the salivary glands and is co-inoculated with the tick saliva in the skin of the vertebrate host. *Borrelia* multiplies at the inoculation site with a peak between day 5 and 15 according to the inoculation route, syringe inoculation or tick bite (Kern et al. 2011 **([3])**). Bacteria migrate extracellularly through the extracellular matrix, enter capillaries, and then spread to distant tissues (Shih et al. 1992 **([4])**). This pathogenesis explains clinical manifestations in human.

Lyme borreliosis is characterized by three clinical phases: First, an early localized inflammation, the erythema migrans, appears in the skin after the tick-infected bite. The second phase, called "early disseminated", is characterized by involvements of the distant organs: joint, nervous system or even the heart. The third, last stage of disease is called "late disseminated", and usually manifests as arthritis or skin disorder known as acrodermatitis chronica atrophians, but can also include neurological manifestations (Stanek et al. 2012 **([5])**). Little is understood about all the process that occurs late in infection and the mechanisms that enable the bacterium to persist, although cellular and humoral immune responses are present. In this context, the role of the skin in the physiopathology of the disease is poorly investigated. Moreover, little is known about how *Borrelia* is acquired by a naïve tick. According to few studies, *Borreliae* present in the dermis of mice either migrate directly to the biting pieces in response to possible chemoattractant or remain indifferent to the tick blood meal (Moriarty et al. 2008 **([6])**; Radolf et al. 2012 **([1])**).

Lyme borreliosis diagnosis is often difficult, especially at late stages of the disease due to the variety of clinical symptoms. Available tests are serology, with sensitivity about 50% in erythema migrans and 97% in acrodermatitis chronica atrophians (Leeflang et al. 2016 **([7])**), PCR and culture (Aguero-Rosenfeld et al. 2005 **([8])**). New diagnostic approaches are therefore required to prove active infection in disseminated manifestations. Selected Reaction Monitoring (SRM) has been recognized as an efficient mass spectrometry-based technique for biomarker detection and validation in several biological fluids (blood, plasma, urine,...) (Domon et Gallien 2015 **([9])**; Kruh-Garcia et al. 2014 **([10])**; Percy et al. 2016 **([11])**; Huttenhain et al. 2012 **([12])**; Kennedy et al. 2014 **([13])**; Percy et al. 2013 **([14])**). However, this approach has never been applied in late infections, nor it has been suggested, to a direct detection of *Borrelia.*

Thus, a need exists of new diagnostic approaches, especially to prove active infection in disseminated manifestations. The present invention fulfills these and other needs.

### Description of the invention

The Applicant has found surprisingly that *Borrelia* presents an organotropism for the skin, which also constitutes a site of persistence in disseminated infections. The Applicant also showed that *Borreliae* found in mouse skin 40 days post-infection were alive, multiply locally and are rapidly infective for unfed ticks.

Then, the Applicant identified several specific borrelial proteins in the late stage of the disease and of a specific reactivation treatment, and developed a proteomic-based approach, which improves late diagnosis for Lyme borreliosis.

Accordingly, in a first aspect, the present invention provides an *ex vivo* method of detecting a *Borrelia burgdorferi* sensu lato active infection in a skin sample of a subject, said skin being treated with an immunosuppressive dermocorticoid before sampling, comprising the step of detecting, in said skin sample, at least one protein of *Borrelia burgdorferi* sensu lato chosen in the group comprising VlsE, flagellin, GAPDH, flagellar filament outer layer protein, L-lactate dehydrogenase, enolase, oligopeptide ABC transporter periplasmic oligopeptide-binding protein (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 (for BbuN40) or gi|365823350 (for BbuB31) or any *Borrelia* species homolog thereof), Hypothetical protein BbuN40_0563 or any *Borrelia* species homolog thereof, and DbpA.

In the present invention, unless otherwise indicated, *"Borrelia burgdorferi" signifies "Borrelia burgdorferi* sensu lato" *or "Borrelia burgdorferi* sl", which covers approximately 20 different species. Among these species, it can be cited for example *Borrelia afzelii, Borrelia burgdorferi* ss (sensu stricto), *Borrelia garinii* and *Borrelia spielmanii.* So, the method of the invention allows detecting *Borrelia burgdorferi* sensu lato in a skin sample, whatever its species. In the case where several species are present in the skin sample, the method makes it possible to identify at least 2 of these species, or even all.

"Active infection" refers herein to an infection by bacteria that are alive, multiply locally and are infective for unfed ticks. The method of the invention is particularly advantageous in detecting active infection of late Lyme borreliosis, i.e. in the late stage of the disease. Indeed in this case, diagnosis is often difficult, especially due to the variety of clinical symptoms.

"Late" Lyme borreliosis, refers herein to the third, last stage of the disease, also called "late disseminated". It usually manifests as arthritis or skin disorder known as acrodermatitis chronica atrophians, but can also include neurological manifestations. When used hereafter, "disseminated" refers to "late disseminated" disease.

The skin sample may be any skin fragment removed from the surface of the body of a subject, comprising at least a fragment of dermis. The skin sample is removed from the body before the implementation of the method of the invention, and stored the necessary time by means of any adapted method known to a person skilled in the art, for example freezing or cultivation. The skin sample may be for example a skin biopsy, as a shave biopsy, a punch biopsy or an excisional biopsy. The skin sample may be derived from any part of the body, as the skin constitutes a site of persistence in late disseminated infections. The skin is treated, at the sampling site, with an immunosuppressive dermocorticoid before the sampling. Advantageously, this treatment allows dermocorticoid to boost locally the bacteria in the skin, and thus to detect them in the skin sample. Without such treatment, the level of bacteria does not allow a detection by proteomic analysis. Thus, the length of treatment of the skin may be sufficient to allow the boost of the bacteria in the skin. It may be for example of 1 to 2 days, for example once or twice a day.

The immunosuppressive dermocorticoid may be a high anti-inflammatory dermocorticoid, a moderate anti-inflammatory dermocorticoid or a low anti-inflammatory dermocorticoid. It may be for example chosen in the group comprising clobetasol, bethamethasone and hydrocortisone.

The subject may be a human or an non-human, i.e. an animal, subject. In case of an animal, it may be any animal capable of contracting or having contracted Lyme disease. It may be for example dogs, horses, cattle or other ruminants.

The *ex vivo* detection, in the skin sample, of the at least one protein, may be realized at the reactivation site, by any mean known by the skilled person. The method may be for example a proteomic method. It may comprise for example a mass spectrometry analysis. It may be for example a gel electrophoresis followed by liquid chromatography-tandem mass spectrometry (Ge-LC-MS/MS) method, or a gel electrophoresis followed by liquid chromatography-selected reaction monitoring-mass spectrometry (Ge-LC-SRM) method, or direct analysis by LC-MS/MS or LC-SRM without gel electrophoresis. Eventually, the detection method may further comprise a quantification analysis of the at least one protein, for example by Ge-LC-SRM assay.

According to the invention, the at least one protein of *Borrelia burgdorferi* sensu lato chosen in the group described above may be at least 1 protein, or at least 2 proteins, or at least 3 proteins, or at least 5 proteins, or at least 10 proteins. For example, it may be 1, or 2, or 3, or 4, or 5, or 6, or 7, or 8, or 9, or 10, or 11, or 12, or 13, or 14 proteins. The invention includes every combination of the cited proteins.

"Protein" refers herein to a native entire protein, or to a peptide, a polypeptide or any homologue thereof, that allows to detect the entire native protein. The skilled person is able to identify the homologous sequences likely to be used according to the invention. For example, a homologous sequence may have at least 80% of homology or identity with the corresponding protein, for example at least 90% or 95%. Methods known by the skilled person may be used to determine the homology or the identity between two ot more sequences, for example the BLAST method (Basic Local Alignment Search Tool). The homologue may be an homologue of *Borrelia* species.

"Comprise" means herein that the invention encompasses the listed proteins, but may also include additional, unnamed proteins. In other words, "comprise" requires it to be interpreted by the broader meaning "include", "contain" or "comprehend". Alternatively, in a particular embodiment of the invention, the at least one protein of *Borrelia burgdorferi* sensu lato may be chosen in the group consisting essentially of the listed proteins, which means that the list of proteins may also include, in addition to the above-mentioned listed proteins, other proteins that do not materially affect the basic and novel characteristics of the invention. In another particular embodiment of the invention, the at least one protein of *Borrelia burgdorferi* sensu lato may be chosen in the group consisting of the listed proteins, which means that the presence of any additional protein is excluded.

For example, the group may comprise VlsE, flagellin and GAPDH.

In another example, the group may comprise flagellar filament outer layer protein, L-lactate dehydrogenase and enolase.

In another example, the group may comprise at least one protein chosen in the group comprising VlsE, flagellin and GAPDH, and at least one protein chosen in the group comprising flagellar filament outer layer protein, L-lactate dehydrogenase, enolase, oligopeptide ABC transporter (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 (for BbuN40) or gi|365823350 (for BbuB31) or any *Borrelia* species homolog thereof, Hypothetical protein BbuN40_0563 or any *Borrelia* species homolog, and DbpA.

For example, the group may comprise at least one protein chosen in the group comprising VlsE, flagellin and GAPDH, and at least one protein chosen in the group comprising flagellar filament outer layer protein, L-lactate dehydrogenase and enolase.

In another example, the group may comprise VlsE, flagellin, GAPDH, flagellar filament outer layer protein, L-lactate dehydrogenase and enolase.

In another example, the group may comprise VlsE, flagellin, GAPDH, flagellar filament outer layer protein, L-lactate dehydrogenase, enolase, oligopeptide ABC transporter (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof), Hypothetical protein BbuN40_0563 or any *Borrelia* species homolog, and DbpA.

The detecting step of at least one protein may be a detection of at least one peptide corresponding to said at least one protein.

The VlsE detection may be a detection of at least one peptide selected in the group comprising:
GIVDAAGTAAGKK (SEQ ID NO: 1),
GIVDAAGTAAGK (SEQ ID NO: 2),
DKVAAALVLR (SEQ ID NO: 3),
VAAALVLR (SEQ ID NO: 4),
AAAAVSSVSGEQILK (SEQ ID NO: 5),
AVSSVSGEQILK (SEQ ID NO: 6),
FSVTNANDANVK (SEQ ID NO: 7),
IGESADNGAAADADSVK (SEQ ID NO: 8),
AAGAVSAVSGEQILK (SEQ ID NO: 9),
FSAADGDGANVK (SEQ ID NO: 10),
AAEAVSSVSGEQILK (SEQ ID NO: 11),
AAEEAIVGATGDGTK (SEQ ID NO: 12),
KDDQIAAAIALR (SEQ ID NO: 13),
DDQIAAAIALR (SEQ ID NO: 14),
AIVDAAGK (SEQ ID NO: 15), and
NPIAAAIGNGNGAGFVDADMR (SEQ ID NO: 16).

The flagellin detection may be a detection of at least one peptide selected in the group comprising:
NNGINAANLSK (SEQ ID NO: 17),
NNAINAANLSK (SEQ ID NO: 18),
AINFIQTTEGNLNEVEK (SEQ ID NO: 19),
ASYAQK (SEQ ID NO: 20),
INAQIR (SEQ ID NO: 21),
DSTEYAIENLK (SEQ ID NO: 22),
LESIKDSTEYAIENLK (SEQ ID NO: 23),
ASDDAAGMGVSGK (SEQ ID NO: 24),
NSTEYAIENLK (SEQ ID NO: 25),
TAEELGMQPAK (SEQ ID NO: 26),
IADQAQYNQMHMLSNK (SEQ ID NO: 27),
ANLGAFQNR (SEQ ID NO: 28),
MKELAVQSGNGTYSDSDR (SEQ ID NO: 29),
ELAVQSGNGTYSDSDR (SEQ ID NO: 30),
INTPASLSGSQASWTLR (SEQ ID NO: 31),
MIINHNTSAINASR (SEQ ID NO: 32) and
GSIQIEIEQLTDEINR (SEQ ID NO: 33).

The GAPDH detection may be a detection of at least one peptide selected in the group comprising:
IIAERDPK (SEQ ID NO: 34),
AVGLVLPELK (SEQ ID NO: 35),
VPVPTGSIVDLTVQLK (SEQ ID NO: 36),
GIDIVAINDLTDPK (SEQ ID NO: 37),
GGYLDHVNHAGAK (SEQ ID NO: 38),
VILTVPAKDEIK (SEQ ID NO: 39),
TIVLGVNDHDINSDLK (SEQ ID NO: 40),
AAALSIIPTSTGAAK (SEQ ID NO: 41),
AVSNASCTTNCLAPLAK (SEQ ID NO: 42),
ILDLPHSDLRR (SEQ ID NO: 43),
ILDLPHSDLR (SEQ ID NO: 44),
VLHESFGIEQGLMTTVHAYTNDQR (SEQ ID NO: 45),
VLSWYDNEFGYSTR (SEQ ID NO: 46) and
LAINGFGR (SEQ ID NO: 47).

The L-lactate dehydrogenase detection is a detection of at least one peptide selected in the group comprising:
DCVNADIVVITAGLNQKPGETR (SEQ ID NO: 48),
GATYYAIGLGIK (SEQ ID NO: 49),
ITEIELDEIHKK (SEQ ID NO: 50),
VIGTGTILDTSR (SEQ ID NO: 51),
IAMKPLSEYLSEGK (SEQ ID NO: 52) and
NIVNAIIGDQNIILPISSYINGQYGGLIK (SEQ ID NO: 53).

The flagellar filament outer layer protein detection may be a detection of at least one peptide selected in the group comprising:
IIKDDVPNYPLASSK (SEQ ID NO: 54),
ISMPEGSFQNFIEK (SEQ ID NO: 55),
YAGDTILGVR (SEQ ID NO: 56),
VYETSGAESLR (SEQ ID NO: 57),
NSVVAPALVK (SEQ ID NO: 58),
AEPGELVLDFAELAR (SEQ ID NO: 59) and
RAEPGELVLDFAELAR (SEQ ID NO: 60).

The enolase detection may be a detection of at least one peptide selected in the group comprising:
GYATSVGDEGGFAPNLK (SEQ ID NO: 61),
SAVPSGASTGINEAVELR (SEQ ID NO: 62),
VNQIGTLTETFEAVEMAK (SEQ ID NO: 63),
SGETEDTTIADLVVALGTGQIK (SEQ ID NO: 64),
VYQYLGAYK (SEQ ID NO: 65) and
IQLVGDDLFVTNTSFLK (SEQ ID NO: 66).

The oligopeptide ABC transporter, periplasmic oligopeptide-binding protein detection is a detection of at least one peptide selected in the group comprising:
ITADAIR (SEQ ID NO: 67),
ALTLAIDR (SEQ ID NO: 68),
DFPIAPIYIYGNSYLFR (SEQ ID NO: 69),
KAEEIIIEK (SEQ ID NO: 70),
FDLSQLK (SEQ ID NO: 71),
YGQSWTNPENIVTSGPFK (SEQ ID NO: 72),
SDYYSSAVNAIYFYAFNTHIKPLDNVK (SEQ ID NO: 73),
NLELFNPEIAK (SEQ ID NO: 74),
TLEITLESPKPYFIDMLVHQSFIPIPIHIAEK (SEQ ID NO: 75),
GLITGDPNTGGNKPGLAK (SEQ ID NO: 76),
ETGSNYSEMVK (SEQ ID NO: 77) and
SWDISPDGTVYTFTLR (SEQ ID NO: 78).

The neutrophil activating protein detection is a detection of at least one peptide selected in the group comprising:
IIDIIAER (SEQ ID NO: 79),
KDDLEEIHSK (SEQ ID NO: 80),
TQDLYEYIEK (SEQ ID NO: 81),
LIDTACDYGTANLIDDIMSDLEK (SEQ ID NO: 82) and
LQELLASLHIFYSNLR (SEQ ID NO: 83).

The 2,3-biphosphoglycerate-dependent phosphoglycerate mutase detection is a detection of at least one peptide selected in the group comprising:
ANDTLNIILK (SEQ ID NO: 84),
LNIPTGIPLVYELDK (SEQ ID NO: 85),
GVDEAIEAGLLLK (SEQ ID NO: 86),
HYGALQGLNK (SEQ ID NO: 87) and
LNIPTGIPLVYELDKDLNPIK (SEQ ID NO: 88).

The elongation factor Tu detection is a detection of at least one peptide selected in the group comprising:
DIDKPFLLAVEDVFSISGR (SEQ ID NO: 89),
TTDVTGVVALEGK (SEQ ID NO: 90),
GSAFGAMSNPEDPEATK (SEQ ID NO: 91),
YGFSANTPIIK (SEQ ID NO: 92),
VGQEVEIVGIK (SEQ ID NO: 93),
GQVLSAPGTITPHKK (SEQ ID NO: 94),
ALKYEDIDNAPEEK (SEQ ID NO: 95),
TTLTAAISIYCSK (SEQ ID NO: 96),
ILEQGQAGDNVGLLLR (SEQ ID NO: 97),
YEDIDNAPEEK (SEQ ID NO: 98) and
HIEYETANR (SEQ ID NO: 99).

The GroEL detection is a detection of at least one peptide selected in the group comprising:
ELLPVLEK (SEQ ID NO: 100),
SALQNAASIAGLLLTTECAITDIKEEK (SEQ ID NO: 101),
QIISNAGFEGSIYIHQIK (SEQ ID NO: 102),
AAVEEGVVPGGGSTLIEVAMYLDTIDTSK (SEQ ID NO: 103),
VDKDNTTIINTGNK (SEQ ID NO: 104),
NVSSGINPIGIK (SEQ ID NO: 105) and
WVNMIESGIIDPAK (SEQ ID NO: 106).

The Lipoprotein gi|256055301 or gi|365823350 detection is a detection of at least one peptide selected in the group comprising:
EISLTPEEAEKLESLK (SEQ ID NO: 107),
SLTEIYSGNGIPLVVSDVVK (SEQ ID NO: 108),
SLTEIDSGNGIPLVVSDVVK (SEQ ID NO: 109),
EFFDWLSK (SEQ ID NO: 110),
GEALSLFFQK (SEQ ID NO: 111) and
VLTESESNNELK (SEQ ID NO: 112).

The Hypothetical protein BbuN40_0563 detection is a detection of at least one peptide selected in the group comprising:
LPLALNIAVFR (SEQ ID NO: 113) and
LPLALNLAVSR (SEQ ID NO: 114).

The DbpA detection is a detection of at least one peptide selected in the group comprising:
TVTDAAEQHPTTTAEGILEIAK (SEQ ID NO: 115),
VSENSFILEAK (SEQ ID NO: 116),
DITDEIDAIK (SEQ ID NO: 117),
GVNFDAFK (SEQ ID NO: 118) and
TTANGIIEIVK (SEQ ID NO: 119).

The method of the invention may be for the diagnosis of late disseminated, also called late Lyme borreliosis, in a subject. The detection of the presence in the skin sample of the at least one protein of *Borrelia burgdorferi* sensu lato as described above allows concluding to the presence of an active infection by *Borrelia burgdorferi* sensu lato, and thus to a late disseminated Lyme borreliosis in the subject.

Another object of the invention relates to a set of proteins of *Borrelia burgdorferi* sensu lato comprising :
- VlsE protein, flagellin protein and GAPDH protein, and eventually,
- at least one protein chosen in the group comprising flagellar filament outer layer protein, L-lactate dehydrogenase, enolase, oligopeptide ABC transporter (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof), Hypothetical protein BbuN40_0563 (or homologs in other *Borrelia* species) and DbpA.

The set of protein may comprise VlsE protein, flagellin protein and GAPDH protein, and at least one polypeptide chosen in the group comprising flagellar filament outer layer protein, L-lactate dehydrogenase, enolase, oligopeptide ABC transporter (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof), Hypothetical protein BbuN40_0563 or any *Borrelia* species homolog and DbpA.

In the set of proteins:
- VlsE protein may be at least one peptide selected in the group comprising:
   GIVDAAGTAAGKK (SEQ ID NO: 1),
   GIVDAAGTAAGK (SEQ ID NO: 2),
   DKVAAALVLR (SEQ ID NO: 3),
   VAAALVLR (SEQ ID NO: 4),
   AAAAVSSVSGEQILK (SEQ ID NO: 5),
   AVSSVSGEQILK (SEQ ID NO: 6),
   FSVTNANDANVK (SEQ ID NO: 7),
   IGESADNGAAADADSVK (SEQ ID NO: 8),
   AAGAVSAVSGEQILK (SEQ ID NO: 9),
   FSAADGDGANVK (SEQ ID NO: 10),
   AAEAVSSVSGEQILK (SEQ ID NO: 11),
   AAEEAIVGATGDGTK (SEQ ID NO: 12),
   KDDQIAAAIALR (SEQ ID NO: 13),
   DDQIAAAIALR (SEQ ID NO: 14),
   AIVDAAGK (SEQ ID NO: 15), and
   NPIAAAIGNGNGAGFVDADMR (SEQ ID NO: 16);
- flagellin protein may be at least one peptide selected in the group comprising:
   NNGINAANLSK (SEQ ID NO: 17),
   NNAINAANLSK (SEQ ID NO: 18),
   AINFIQTTEGNLNEVEK (SEQ ID NO: 19),
   ASYAQK (SEQ ID NO: 20),
   INAQIR (SEQ ID NO: 21),
   DSTEYAIENLK (SEQ ID NO: 22),
   LESIKDSTEYAIENLK (SEQ ID NO: 23),
   ASDDAAGMGVSGK (SEQ ID NO: 24),
   NSTEYAIENLK (SEQ ID NO: 25),
   TAEELGMQPAK (SEQ ID NO: 26),
   IADQAQYNQMHMLSNK (SEQ ID NO: 27),
   ANLGAFQNR (SEQ ID NO: 28),
   MKELAVQSGNGTYSDSDR (SEQ ID NO: 29),
   ELAVQSGNGTYSDSDR (SEQ ID NO: 30),
   INTPASLSGSQASWTLR (SEQ ID NO: 31),
   MIINHNTSAINASR (SEQ ID NO: 32) and
   GSIQIEIEQLTDEINR (SEQ ID NO: 33);
- GAPDH protein may be at least one peptide selected in the group comprising:
   IIAERDPK (SEQ ID NO: 34),
   AVGLVLPELK (SEQ ID NO: 35),
   VPVPTGSIVDLTVQLK (SEQ ID NO: 36),
   GIDIVAINDLTDPK (SEQ ID NO: 37),
   GGYLDHVNHAGAK (SEQ ID NO: 38),
   VILTVPAKDEIK (SEQ ID NO: 39),
   TIVLGVNDHDINSDLK (SEQ ID NO: 40),
   AAALSIIPTSTGAAK (SEQ ID NO: 41),
   AVSNASCTTNCLAPLAK (SEQ ID NO: 42),
   ILDLPHSDLRR (SEQ ID NO: 43),
   ILDLPHSDLR (SEQ ID NO: 44),
   VLHESFGIEQGLMTTVHAYTNDQR (SEQ ID NO: 45),
   VLSWYDNEFGYSTR (SEQ ID NO: 46) and
   LAINGFGR (SEQ ID NO: 47);
- DbpA protein may be at least one peptide selected in the group comprising:
   TVTDAAEQHPTTTAEGILEIAK (SEQ ID NO: 115),
   VSENSFILEAK (SEQ ID NO: 116),
   DITDEIDAIK (SEQ ID NO: 117),
   GVNFDAFK (SEQ ID NO: 118) and
   TTANGIIEIVK (SEQ ID NO: 119);
- L-lactate dehydrogenase protein may be at least one peptide selected in the group comprising:
   DCVNADIVVITAGLNQKPGETR (SEQ ID NO: 48),
   GATYYAIGLGIK (SEQ ID NO: 49),
   ITEIELDEIHKK (SEQ ID NO: 50),
   VIGTGTILDTSR (SEQ ID NO: 51),
   IAMKPLSEYLSEGK (SEQ ID NO: 52) and
   NIVNAIIGDQNIILPISSYINGQYGGLIK (SEQ ID NO: 53);
- flagellar filament outer layer protein may be at least one peptide selected in the group comprising:
   IIKDDVPNYPLASSK (SEQ ID NO: 54),
   ISMPEGSFQNFIEK (SEQ ID NO: 55),
   YAGDTILGVR (SEQ ID NO: 56),
   VYETSGAESLR (SEQ ID NO: 57),
   NSVVAPALVK (SEQ ID NO: 58),
   AEPGELVLDFAELAR (SEQ ID NO: 59) and
   RAEPGELVLDFAELAR (SEQ ID NO: 60);
- enolase protein may be at least one peptide selected in the group comprising:
   GYATSVGDEGGFAPNLK (SEQ ID NO: 61),
   SAVPSGASTGINEAVELR (SEQ ID NO: 62),
   VNQIGTLTETFEAVEMAK (SEQ ID NO: 63),
   SGETEDTTIADLVVALGTGQIK (SEQ ID NO: 64),
   VYQYLGAYK (SEQ ID NO: 65) and
   IQLVGDDLFVTNTSFLK (SEQ ID NO: 66);
- oligopeptide ABC transporter (for example gi|111115153 or any *Borrelia* species homolog thereof) may be at least one peptide selected in the group comprising:
   ITADAIR (SEQ ID NO: 67),
   ALTLAIDR (SEQ ID NO: 68),
   DFPIAPIYIYGNSYLFR (SEQ ID NO: 69),
   KAEEIIIEK (SEQ ID NO: 70),
   FDLSQLK (SEQ ID NO: 71),
   YGQSWTNPENIVTSGPFK (SEQ ID NO: 72),
   SDYYSSAVNAIYFYAFNTHIKPLDNVK (SEQ ID NO: 73),
   NLELFNPEIAK (SEQ ID NO: 74),
   TLEITLESPKPYFIDMLVHQSFIPIPIHIAEK (SEQ ID NO: 75),
   GLITGDPNTGGNKPGLAK (SEQ ID NO: 76),
   ETGSNYSEMVK (SEQ ID NO: 77) and
   SWDISPDGTVYTFTLR (SEQ ID NO: 78)
- neutrophil activating protein protein may be at least one peptide selected in the group comprising:
   IIDIIAER (SEQ ID NO: 79),
   KDDLEEIHSK (SEQ ID NO: 80),
   TQDLYEYIEK (SEQ ID NO: 81),
   LIDTACDYGTANLIDDIMSDLEK (SEQ ID NO: 82) and
   LQELLASLHIFYSNLR (SEQ ID NO: 83);
- 2,3-biphosphoglycerate-dependent phosphoglycerate mutase protein may be at least one peptide selected in the group comprising:
   ANDTLNIILK (SEQ ID NO: 84),
   LNIPTGIPLVYELDK (SEQ ID NO: 85),
   GVDEAIEAGLLLK (SEQ ID NO: 86),
   HYGALQGLNK (SEQ ID NO: 87) and
   LNIPTGIPLVYELDKDLNPIK (SEQ ID NO: 88);
- elongation factor Tu protein may be at least one peptide selected in the group comprising:
   DIDKPFLLAVEDVFSISGR (SEQ ID NO: 89),
   TTDVTGVVALEGK (SEQ ID NO: 90),
   GSAFGAMSNPEDPEATK (SEQ ID NO: 91),
   YGFSANTPIIK (SEQ ID NO: 92),
   VGQEVEIVGIK (SEQ ID NO: 93),
   GQVLSAPGTITPHKK (SEQ ID NO: 94),
   ALKYEDIDNAPEEK (SEQ ID NO: 95),
   TTLTAAISIYCSK (SEQ ID NO: 96),
   ILEQGQAGDNVGLLLR (SEQ ID NO: 97),
   YEDIDNAPEEK (SEQ ID NO: 98) and
   HIEYETANR (SEQ ID NO: 99);
- GroEL may be at least one peptide selected in the group comprising:
   ELLPVLEK (SEQ ID NO: 100),
   SALQNAASIAGLLLTTECAITDIKEEK (SEQ ID NO: 101),
   QIISNAGFEGSIYIHQIK (SEQ ID NO: 102),
   AAVEEGVVPGGGSTLIEVAMYLDTIDTSK (SEQ ID NO: 103),
   VDKDNTTIINTGNK (SEQ ID NO: 104),
   NVSSGINPIGIK (SEQ ID NO: 105) and
   WVNMIESGIIDPAK (SEQ ID NO: 106);
- Lipoprotein gi|256055301 or gi|365823350 (or any *Borrelia* species homolog thereof) is at least one peptide selected in the group comprising:
   EISLTPEEAEKLESLK (SEQ ID NO: 107),
   SLTEIYSGNGIPLVVSDVVK (SEQ ID NO: 108),
   SLTEIDSGNGIPLVVSDVVK (SEQ ID NO: 109),
   EFFDWLSK (SEQ ID NO: 110),
   GEALSLFFQK (SEQ ID NO: 111) and
   VLTESESNNELK (SEQ ID NO: 112);
- Hypothetical protein BbuN40_0563 may be at least one peptide selected in the group comprising:
   LPLALNIAVFR (SEQ ID NO: 113) and
   LPLALNLAVSR (SEQ ID NO: 114)
- DbpA may be at least one peptide selected in the group comprising:
   TVTDAAEQHPTTTAEGILEIAK (SEQ ID NO: 115),
   VSENSFILEAK (SEQ ID NO: 116),
   DITDEIDAIK (SEQ ID NO: 117),
   GVNFDAFK (SEQ ID NO: 118) and
   TTANGIIEIVK (SEQ ID NO: 119).

Another object of the invention relates to a kit for detecting *Borrelia burgdorferi* sensu lato in a subject, comprising at least one immunosuppressive dermocorticoid as defined above, and a set of polypeptides as defined above.

Another object of the invention relates to the use of a set of polyeptides as defined above, in an *ex vivo* method of detecting *Borrelia burgdorferi* sensu lato in a subject likely to present an active Lyme infection.

A method for reactivating *in vivo Borrelia burgdorferi* sensu lato active infection in a skin sample of a subject, comprising administering at least one immunosuppressive dermocorticoid to the skin of said subject, is described.

A method of diagnosis of a *Borrelia burgdorferi* sensu lato active infection in a subject, comprising the steps of:
- administering at least one immunosuppressive dermocorticoid to the skin of said subject, and
- detecting, in said skin sample, at least one protein of *Borrelia burgdorferi* sensu lato chosen in the group comprising VlsE, flagellin, GAPDH, flagellar filament outer layer protein, L-lactate dehydrogenase, enolase, oligopeptide ABC transporter periplasmic oligopeptide-binding protein (for example gi|111115153 or any *Borrelia* species homolog thereof), neutrophil activating protein, 2,3-biphosphoglycerate-dependent phosphoglycerate mutase, elongation factor Tu, GroEL, Lipoprotein gi|256055301 (or gi|365823350 or any *Borrelia* species homolog thereof), Hypothetical protein BbuN40_0563 or any *Borrelia* species homolog thereof, and DbpA
is described.

This invention is further illustrated by the following examples with regard to the annexed drawings that should not be construed as limiting.

### Brief description of the figures

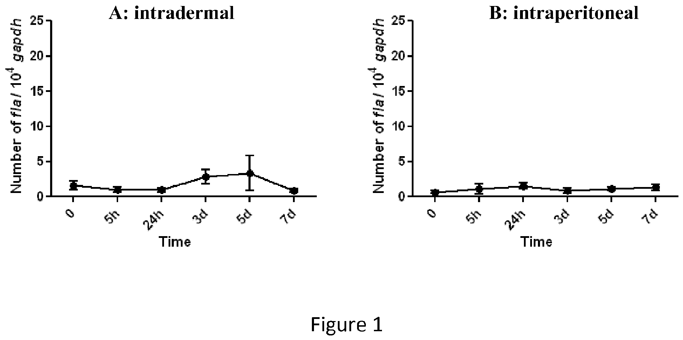

- Figure 1 represents the effect of uninfected tick bite on *Borrelia* present in the skin. Detection of *Borrelia* in the skin by quantitative PCR, 40 days after initial intradermal (A) or intraperitoneal (B) inoculations. Different distant organs were tested by culture and/or PCR to measure the presence of *Borrelia.* The blood was also tested and remained negative during all the process of the tick blood meal. Five ticks were fed on each uninfected mouse. The tick bite is not sufficient to get a local reactivation in the skin.

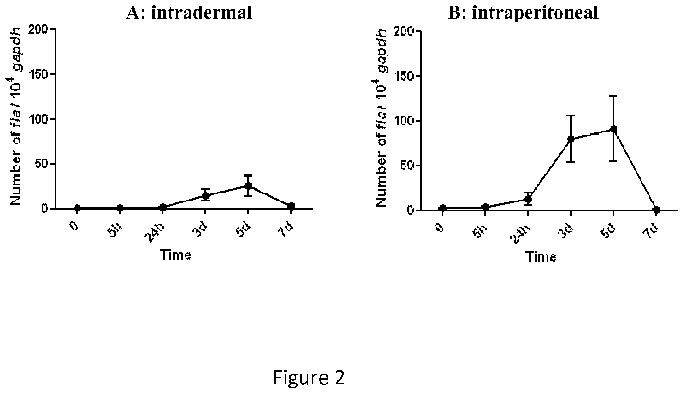

- Figure 2 represents the effect of the dermocorticosteroid, clobetasol on Borrelia present in the skin. Detection of Borrelia in the skin by quantitative PCR, 40 days after initial intradermal (A) or intraperitoneal (B) inoculations. Different distant organs were tested by culture and/or PCR to measure the presence of Borrelia. The blood was also tested and remained negative during all the process of the tick blood meal, except for two animals intraperitonally inoculated. The dermocorticoid, by its immunosuppressive effect on the skin, is able to reactivate the bacteria locally.

### EXAMPLES

To better understand the role of the skin in disseminated infections, we inoculated *Borrelia* by different routes, intradermally or intraperitoneally or via infected ticks. To better reactivate *Borrelia* locally, we applied an immunosuppressive dermocorticoid, allowing the detection of a significant number of bacteria by PCR. We then undertook a proteomic analysis on mouse skin, using gel electrophoresis followed by liquid chromatography and tandem mass spectrometry (Ge-LC-MS/MS) to identify bacterial proteins. Indeed, we have shown recently that this method is efficient to identify *Borrelia* in the skin early after the transmission (Schnell et al. 2015 **([15])**). These proteins might constitute candidates for markers of active infection in disseminated infections in patients with Lyme borreliosis. Then we set up a Ge-LC-SRM assay to specifically detect these protein markers. The opportunity to develop a new diagnosis using these markers is discussed.

### Materials and Methods

### Animals, Borrelia and ticks

C3H/HeN mice were purchased from Charles River Laboratories. *B*. *burgdorferi* sensu stricto (ss) N40; *B*. *afzelii* NE4049 and *B*. *garinii* PBi were cultured in BSK-H complete medium (Sigma) at 33°C and used at low passage for mouse infections.

The protocols carried out in this study were approved by the CREMEAS Committee on the Ethics of Animal Experiments of the University y of Strasbourg (Comité Régional d'Ethique en Matière d'Expérimentation Animale Strasbourg- Permit Number: 2015062210282757- National guidelines follow EEC Council Directive 2010/63/EU).

### Infections of mice with Borrelia burgdorferi sl

Mice (3-4 weeks old) were inoculated intradermally in the dorsal thoracic area (n=73) or intraperitoneally (n=63) via syringe with 10³/100µL *B*. *burgdorferi* ss N40, *B*. *afzelii* NE4049 or *B*. *garinii* PBi (counted by Petroff-Hausser chamber). After 2 to 3 weeks, mouse sera were tested by ELISA IgG, as described by Kern et al. **([3])**. Mice infected through tick bite were infected by 5 or 10 nymphs as described by Mbow et al. (Mbow et al. 1994 **([16])**). Ticks positive for *B*. *afzelii* NE4049 were previously infected at the larval stage according to the protocol of Tonetti et al. (Tonetti et al. 2015 **([17])**).

### Transmission of Borrelia from infected mice to naïve ticks.

Five naïve ticks were fed on infected animals as previously described (Kern et al. **([3])**) after around 40 days of infection. After detachment, ticks were collected and tested by PCR to detect *Borrelia.* For *B*. *burgdorferi* detection by qualitative PCR, DNA was extracted from the ticks on a MagNA Pure apparatus as described by the manufacturer (Roche Diagnostics). Qualitative amplification targeting the *fla* gene was carried out as described (Jaulhac et al. 2000 **([18])**).

### Borrelia reactivation by dermocorticoid applications

Around 10 mg of Dermoval® (0.05% clobetasol) were applied in the dorsal thoracic area, twice a day for 2 days, 40 days after the initial infection. After reactivation, mice were killed by Forene® inhalation (isoflurane) at different times (0, 5h, 24h, 3d, 5d, 7d). For each mouse at each time point, different organs were collected (heart, joints, dorsal thoracic skin and blood). A minimum of 5 mice was used at each time point (maximum 13).

### Borrelia-infected animals, reactivated with dermocorticoid and treated with antibiotics

Forty days after the infection with 10³/100 µL *B*. *burgdorferi* ss N40 intradermally, the bacteria were first reactivated with clobetasol, twice a day for 2 days, then mice were treated with antibiotics (ceftriaxone 16 mg/kg) intradermally, twice a day for 5 days. At day 1, 2, 3 and 7, the skin was collected and cultured for *Borrelia* detection, or frozen at -80°C for PCR quantification and proteomics analyses. Negative controls correspond to infected animals treated with clobetasol, then inoculated with saline solution.

### Culture detection of B. burgdorferi sensu lato in organs or blood

We dissected aseptically different organs and transferred them into BSK-H medium at 33°C for 5 weeks. PCR was performed only if culture was negative or contaminated. For *Borrelia* detection by culture, dissected heart, joint, blood (3 drops) and dorsal thoracic skin were transferred in BSK-H medium at 33°C and examined weekly during 5 weeks by dark-field microscopy to detect the presence of spirochetes.

### Borrelia quantification in mouse skin by PCR

Samples were tested as described previously (Kern et al. **([3])**). For *B*. *burgdorferi* detection by quantitative PCR, DNA was extracted from the skin of individual mice on a MagNA Pure apparatus as described by the manufacturer (Roche Diagnostics). Qualitative amplification targeting the *fla* gene was carried out. Quantification of the *B*. *burgdorferi-*specific *fla* gene was performed on a LightCycler system (Roche Diagnostics). Quantification of the mouse specific *gapdh* gene was done on an ABI Prism 7000 instrument (Applied Biosystem), using a commercial kit (TaqMan rodent GADPH control reagent; Applied Biosystem). Number of *B*. *burgdorferi* spirochetes in tissue specimens was normalized to 10⁴ *gapdh* DNA copies.

### Ge-LC-MS/MS analyses

For non-targeted proteomics, samples (5 mg) of mouse skin biopsies were manually extracted by Laemmli buffer and proteins (50 µg) were pre-fractionated onto 12% SDS-PAGE as described (Schnell et al. **([15])**). Gel bands (10 ± 1 bands) of 2 mm were excised manually. After reduction, alkylation and trypsin digestion, the peptides were extracted using 80% ACN and 0.1% HCOOH for 90 min at room temperature. After evaporation, the peptides were suspended in 50 µL of 0.1 % HCOOH prior to mass spectrometry analyses. LC-MS/MS analyses were performed on a nanoACQUITY Ultra-Performance-LC system hyphenated to a Q-Exactive Plus mass spectrometer as described (Muller et al. 2016 **([19])**). Data analysis was performed as described **([15])** against an in-house database containing all protein sequences of *Borrelia* and mouse extracted from NCBlnr and UniProtKB-SwissProt, respectively. For the bacteria, four different reference databases were used depending on the strain analysed: *B*. *burgorferi* ss B31 (1758 entries at May 07, 2013), *B*. *burgdorferi* ss N40 (1480 entries at January 30, 2015), *B*. *afzelii* Pko (2180 entries at October 16, 2014), and *B*. *garinii* Pbi (1410 entries at April 08, 2015). For MS² data, parent and fragment mass tolerance was 5 ppm and 0.07 Da and a maximum of 1 missed cleavage was allowed. The Mascot and OMSSA results were independently loaded into the Proline software (Proline Studio Release 1.0). All spectra leading to an identification exceeding a minimum set threshold (Mascot Ion Score > 25, OMSSA -log(e-value) > 7, peptide length > 6 amino acids) and having a pretty rank as defined by Mascot equal to 1 were kept. Resulting spectra were then filtered to obtain a protein false discovery rate of less than 1% (Elias et al. 2007 **([20])**).

### Ge-LC-SRM analyses

For targeted proteomics, protein fractionation and *in gel* digestion was achieved as previously described for Ge-LC-MS/MS analyses with the following modifications. First, two different protein quantities, 100 µg and 75 µg, were electrophoresed onto SDS-PAGE to obtain respectively 16 and 11 bands. Second, after protein digestion, extraction and evaporation, the peptides (average of 6.25 µg of proteins per gel band) were suspended in 6 µL of a mixture of diluted heavy labelled peptides (Thermo Fisher) prior to mass spectrometry.

For each band, the totality of the suspended peptide mixture (6 µL) was injected. For LC-SRM assay, 67 proteotypic peptides were selected for 14 targeted proteins (DbpA, Enolase, Flagellin, GAPDH, HSP90, BB0081, BBP42, BAPKO0593, BAPKO4515, lipoprotein gi|365823350, VlsE, RNA polymerase, Histidine kinase, hypothetical protein gi|500023077) and isotopically labelled equivalent peptides were purchased (64 crude PEPotecs peptides and three high purity AQUA® flagellin peptides). Four transitions per peptide corresponding to the most abundant y or b mono- or doubly charged ions were selected for both endogenous and heavy labelled peptides. For each transition, the collision energy was optimized experimentally by testing six values centred on the reference value. The reference value is calculated using the equation given by the supplier. A total of 544 transitions corresponding to 136 precursors were monitored. For the SRM analyses, 6 µL of a mixture of diluted heavy labelled peptides was added to suspend an average of 6.25 µg peptides (100 µg of proteins / 16 gel bands or 75 µg of proteins / 11 gel bands).

All separations were carried out on Agilent 1100 Series HPLC system (Agilent Technologies). For each analysis, the sample was loaded into a trapping column ZORBAX 300SB-C18 MicroBore Guard 5 µm, 1.0 x 17 mm (Agilent Technologies) at 50 µL/min with an aqueous solution containing 0.1% HCOOH and 2% ACN (solvent A). After 3 min of trapping, the column was put on-line with a ZORBAX 300SB-C18 3.5 µm, 0.3 x 150 mm column (Agilent Technologies). Peptide elution was performed at 5 µL/min using a gradient from 5-45% solvent B (ACN in 0.1% HCOOH) over 57 min. The isolation width for both Q1 and Q3 was set to 0.7 m/z unit. Time-scheduled SRM method targeted the pairs of isotopically labelled peptides/endogenous peptides in ± 3 min retention time windows (6 min in total) within a cycle time of 3000 ms. Mass data collected during LC-SRM were processed with the Skyline open-source software package 3.5.9.

### Results

### B. burgdorferi ss N40 disseminates similarly after intradermal and intraperitoneal inoculation 40 days of infection.

To demonstrate the role of the skin as homing and persistence site for *Borrelia* in disseminated infections, we tested two inoculation routes, intradermal and intraperitoneal. The presence of *Borrelia* was tested by culture and / or PCR in different tissues (blood, joints, distant skin and heart), 40 days after *Borrelia* inoculation (Table 1). The sites of persistence of bacteria in late phases of the disease are the same whatever the route of inoculation: the heart, the joint and the skin or the ear. The blood culture is negative for both. Interestingly, after intraperitoneal inoculation, the skin of the ear is strongly positive although, it has been bypassed, demonstrating the role of the skin as homing site in Lyme borreliosis.

**Table 1. Culture of blood and organs of C3H/HeN mice at day 40, after inoculation either intradermally or intraperitonally of 10³/100 µL Borrelia burgdorferi ss N40.**

| Inoculation protocol | Blood | Heart | Joint | Ear skin | Serology |
|---|---|---|---|---|---|
| Intradermal | 0% (0/13) | 62% (8/13) | 92% (12/13) | 92% (12/13) | 100% (13/13) |
| Intraperitoneal | 0% (0/9) | 66% (6/9) | 100% (9/9) | 100% (9/9) | 100% (9/9) |

### Transmission of B. burgdorferi ss N40 to naive ticks in persistent Lyme infection.

We compared the transmission of *Borrelia* from mice previously infected either intradermally or intraperitoneally to naïve ticks. Tick saliva has been described as immunosuppressive during *Borrelia* inoculation to naïve mice (Kazimírová and Stibrániová 2013 **([21])**). We tested whether tick saliva reactivates *Borrelia* in infected animals. To this end, five ticks were fed on the back of each infected mouse and at different time points after the blood meal, ticks were collected and checked by PCR for the presence of *Borrelia* (Table 2). Interestingly, the transmission of the bacteria occurs rapidly 5 hours after intradermal inoculation and 8h after intraperitoneal inoculation. Then, positivity increases over time up to 70 to 80%. The rate of infection in ticks is higher and quicker after intradermal inoculation (Table 2).

**Table 2. Positivity of ticks fed at different time points on mice infected by Borrelia burgdorferi ss N40 40 days ago, either intradermally or intraperitoneally.**

| | **Intradermal** | | **Intraperitoneal** | |
|---|---|---|---|---|
| **Time after tick deposit** | **Contamination rate of tick (%)** | **Number of ticks tested** | **Contamination rate of tick (%)** | **Number of ticks tested** |
| 5h | 22 | 9 | 0 | 12 |
| 8h | 80 | 15 | 6.25 | 16 |
| 24h | 72 | 18 | 6.25 | 16 |
| 3d | 50 | 28 | 5 | 21 |
| 5d | 100 | 11 | 70 | 10 |
| 7d | 86 | 7 | 71 | 7 |

No significant reactivation of bacteria was observed after tick bite in the skin as shown by PCR (Figure 1A and B). During the blood meal of uninfected ticks, the uptake of *Borrelia* is not linked to the reactivation in distant organs, since over the course of the blood meal, the blood remains negative although all the organs are positive (Table 3 and Table 4).

**Table 3.**

| **C** | **Time after tick bite** | **Blood (%)** | **Heart (%)** | **Joint (%)** | **Ear skin (%)** |
|---|---|---|---|---|---|
| **Intradermal** | 0 | 0 | 61,5 | 92 | 92 |
| | 5h | 0 | 60 | 80 | 100 |
| | 24h | 0 | 100 | 100 | 100 |
| | 3d | 12,5 (1/8) | 75 | 87,5 | 87,5 |
| | 5d | 0 | 80 | 80 | 100 |
| | 7d | 0 | 40 | 100 | 80 |

**Table 4.**

| | **Time after tick bite** | **Blood (%)** | **Heart (%)** | **Joint (%)** | **Ear skin (%)** |
|---|---|---|---|---|---|
| **Intraperitoneal** | 0 | 0 | 66,6 | 100 | 100 |
| | 5h | 0 | 60 | 80 | 100 |
| | 24h | 0 | 100 | 100 | 100 |
| | 3d | 0 | 57,1 | 100 | 100 |
| | 5d | 0 | 60 | 100 | 100 |
| | 7d | 0 | 60 | 100 | 100 |

### Reactivation of Borrelia in mouse skin after dermocorticoid application.

To demonstrate the presence of live *Borreliae* in the skin and improve the bacterial detection and quantification by PCR, we applied a local immunosuppressive drug, a dermocorticoid, to the mouse skin. Application of two days of clobetasol reactivated the bacteria specifically in the skin and better after intraperitoneal infection (figure 2A and B). The rise of bacteria begun 24 hours after the beginning of the application and a maximum was observed at day 3 and day 5, to then decrease to a basal rate at day 7. In the intradermal protocol, a similar pattern was observed. Among the skin tested by PCR, 92.5% were positive in culture (n =45 for A and n=36 for B), indicating that the observed increase came of well live bacteria (Table 5 and Table 6).

**Table 5.**

| **C** | **Time after corticoid reactivation** | **Blood (%)** | **Heart (%)** | **Joint (%)** | **Ear skin (%)** |
|---|---|---|---|---|---|
| **Intradermal** | 0 | 0 | 61.5 | 92.3 | 92.3 |
| | 5h | 0 | 80 | 100 | 60 |
| | 24h | 0 | 80 | 100 | 60 |
| | 3d | 0 | 80 | 100 | 100 |
| | 5d | 0 | 100 | 100 | 83.3 |
| | 7d | 0 | 50 | 100 | 100 |

**Table 6.**

| **D** | **Time after corticoid reactivation** | **Blood (%)** | **Heart (%)** | **Joint (%)** | **Ear skin (%)** |
|---|---|---|---|---|---|
| **Intraperitoneal** | 0 | 0 | 66.6 | 100 | 100 |
| | 5h | 0 | 100 | 100 | 100 |
| | 24h | 20 (1/5) | 60 | 100 | 100 |
| | 3d | 0 | 83.3 | 100 | 100 |
| | 5d | 0 | 83.3 | 100 | 100 |
| | 7d | 20(1/5) | 100 | 100 | 100 |

Although we used a dermocorticoid that reactivates *Borrelia* in the skin, the blood remained negative after intradermal inoculation and scarcely positive after intraperitoneal inoculation. We conclude that after dissemination, *Borreliae* clearly persist in the skin as live bacteria and in absence of corticosteroid, the level is sufficient to assure an efficient transmission to naïve ticks. The dermocorticoid induced the local multiplication of live *Borreliae.*

### Identification of Borrelia burgdorferi ss N40 proteins in mouse skin by Ge-LC/MS-MS during late infection after intraperitoneal inoculation

We have shown previously that Ge-LC-MS/MS strategy can be efficient to detect *Borrelia* proteins, in the skin of infected mice early after the transmission of bacteria, during their peak of multiplication (Schnell et al. **([15])**). When mice were infected intradermally with *B*. *burgdorferi* ss N40, the infection rate was very low (≤ 2 *fla*/10⁴ *gapdh*) after disseminated infection. Flagellin was the single protein identified with confidence in one mouse over 5 analyzed (data not shown). Since we showed that intraperitoneal infection and clobetasol reactivation led to a peak of multiplication of *Borrelia* in the skin (Figure 2D), we analyzed the proteins present in the skin of 6 mice with a significant infection rate (from 90 to 259 *fla*/10⁴ *gapdh*). As expected, we identified mostly mouse proteins (up to 3761). However, we also detected a total of 8 *Borrelia* proteins among which flagellin, VlsE, DbpA and GAPDH (Table 7). A PCR quantification with high flagellin rate led to several bacterial proteins: 7 proteins in mouse #6 with 259 *fla*/10⁴ *gapdh.*

**Table 7. Identification of bacterial proteins by Ge-LC-MS/MS in mouse skin infected intraperitonally with Borrelia burgdorferi ss N40 and reactivated with clobetasol, 40 days after initial inoculation. Proteins were identified using both Mascot and OMSSA algorithms. Numbers of identified peptides are bracketed.**

| | **Mouse 1** | **Mouse 2** | **Mouse 3** | **Mouse 4** | **Mouse 5** | **Mouse 6** |
|---|---|---|---|---|---|---|
| **Infection rate *fla*/10⁴ *gapdh*** | *119* | *103* | *203* | *90* | *99* | *259* |
| **Number of identified mouse proteins** | 3733 | 3761 | 3723 | 3552 | 3077 | 3373 |
| **Flagellin** | X (5) | X (1) | X (5) | | X (2) | X (4) |
| **VlsE** | X (3) | X (4) | X (6) | | X (1) | X (6) |
| **DbpA** | X (1) | X (1) | | | | |
| **GAPDH** | | X (1) | | | | X (1) |
| **Hypothetical protein BbuN40_0563** | | | | | | X (1) |
| **Lipoprotein gi\|256055301** | | | | | | X (1) |
| **Chaperonin GroEL** | | | | | | X (1) |
| **Elongation factor Tu** | | | | | | X (1) |

### Identification of proteins of different species of Borrelia in mouse skin after intradermal inoculation

*Borrelia* is inoculated through the dermis upon dilacerations of host skin by tick mouthparts. Since intradermal inoculation mimics better the process of tick infection, we tested the intradermal route for the three most pathogenic species of *Borrelia* in humans: *B*. *burgdorferi* ss, *B*. *afzelii* and *B*. *garinii. B*. *afzelii* gave the best multiplication in mouse skin after cortisone reactivation as shown with PCR quantification (*fla*/10⁴ *gapdh*) (Table 8). We detected 3 *Borrelia* proteins: VlsE, flagellin and GAPDH among more than 3 000 mouse proteins. The two other *Borrelia* species multiplied less efficiently in the skin compared to *B*. *afzelii.*

**Table 8. Identification of bacterial proteins by Ge-LC-MS/MS in mouse skin infected intradermally with different species of B. burgdorferi sensu lato and reactivated with clobetasol, 40 days after the initial inoculation. Proteins were identified using both Mascot and OMSSA algorithms. Numbers of identified peptides are bracketed.**

| | ***B. burgdorferi* ss N40** | | | ***B. afzelii* NE4049** | | | | | | | ***B. garinii* PBi** | | |
|---|---|---|---|---|---|---|---|---|---|---|---|---|---|
| | Mouse 1 | Mouse 2 | Mouse 3 | Mouse 1 | Mouse 2 | Mouse 3 | Mouse 4 | Mouse 5 | Mouse 6 | Mouse 7 | Mouse 1 | Mouse 2 | Mouse 3 |
| ***Infection rate fla*/*a10⁴ gapdh*** | *24* | *10* | *43* | *304* | *151* | *3* | *69* | *92* | *38* | *112* | *14* | *30* | *19* |
| **Number of identified mouse proteins** | 3302 | 3458 | 3482 | 3440 | 3147 | 3580 | 3513 | 3009 | 2979 | 3077 | 3381 | 3374 | 3279 |
| **Flagellin** | | | | X (4) | | | X (3) | | | | | | |
| **VlsE** | | | X (3) | X (1) | | | | X (1) | | X (1) | | | |
| **GAPDH** | | | | X (2) | | | | | | | | | |

### Identification of Borrelia afzelii proteins in mouse skin after infectious tick bite

We have previously shown that infected nymphs inoculate a very small amount of bacteria during the blood meal. Since *B*. *afzelii* multiplies most efficiently than the two other *Borrelia* species tested, we used this species to analyze reactivation of *Borrelia* in the skin after a tick bite. To detect sufficient amount of bacterial proteins, we infected mice with 10 infected ticks. Surprisingly with 10 infected ticks, we got very good transmission and detection of *Borrelia* proteins (Table 9). Ten *Borrelia* proteins were identified. As after needle inoculation, flagellin, VlsE and GAPDH were detected. Some were specifically detected after tick transmission: flagellar filament outer layer protein, L-lactate dehydrogenase, enolase, oligopeptide ABC transporter, neutrophil activating protein and 2,3-biphosphoglycerate-dependent phosphoglycerate mutase.

**Table 9. Identification of bacterial proteins by Ge-LC-MS/MS in mouse skin infected via ticks with Borrelia afzelii NE4049 and reactivated with clobetasol, 40 days after initial inoculation. Among the 36 proteins identified with both Mascot and OMSSA algorithms, only those identified with at least 3 peptides in one biopsy are listed. Number of identified peptides are bracketed.**

| | **Mouse 1** | **Mouse 2** | **Mouse 3** | **Mouse 4** |
|---|---|---|---|---|
| ***Infection rate fla*/*10⁴ gapdh*** | *876* | *602* | *1231* | *723* |
| **Number of identified mouse proteins** | 4489 | 4156 | 3961 | 4372 |
| **Flagellin** | X (11) | X (8) | X (8) | X (10) |
| **Flagellar filament outer layer protein** | X (1) | X (4) | X (1) | X (6) |
| **VlsE** | X (3) | X (2) | X (2) | X (2) |
| **L-lactate dehydrogenase** | X (3) | X (5) | | X (4) |
| **GAPDH** | X (4) | X (5) | X (7) | X (6) |
| **Enolase** | X (3) | X (1) | | X (2) |
| **Oligopeptide ABC transporter, gi\|111115153** | X (3) | X (2) | X (1) | X (6) |
| **Neutrophil activating protein** | X (3) | X (1) | | |
| **2,3-biphosphoglycerate-dependent phosphoglycerate mutase** | X (2) | X (1) | X (2) | X (3) |
| **Elongation factor Tu** | X (3) | X (7) | X (2) | X (9) |

We also analyzed skin biopsies after infection by 5 infected ticks. The infection rate was lower and no protein was identified using both Mascot and OMSSA algorithms. Some proteins were identified by a single algorithm (data not shown) but were not different from the ones identified previously.

### Detection of specific Borrelia proteins in infected mouse skin by targeted proteomics (Ge-LC-SRM)

To improve the detection and quantify *Borrelia* proteins in mouse skin, we set up targeted proteomics. Among all *Borrelia* proteins identified by Ge-LC-MS/MS analyses, 14 proteins were selected to set up a targeted Ge-LC-SRM assay. The method monitored 544 transitions corresponding to 67 peptides. According to *Borrelia* proteins variation, the selection of proteotypic peptides was done by including a selection of a large set of variants, especially for flagellin and VlsE proteins. The specificity and sensitivity of Ge-LC-SRM allowed to investigate the detection of *Borrelia* proteins at the late stage of infection in mouse skin biopsies with lower infection rate than for Ge-LC-MS/MS. To assess our Ge-LC-SRM detection method, we analyzed two skin biopsies, infected either by *B*. *burgdorferi* ss N40 or *B*. *afzelii* NE4049 with high PCR quantification (259 and 304 *fla*/10⁴ *gapdh* respectively). After extraction, 75 µg of protein extract were loaded onto the gel (16 bands) and analyzed by LC-SRM. The detection of a peptide was validated as we observed (1) the co-elution for all transitions, (2) the co-elution between heavy labeled and the endogenous peptide, (3) a consistent ratio between peptide transitions for endogenous- and heavy labeled peptides and (4) a signal greater than 3 times the signal to noise ratio. Several *Borrelia* proteins/peptides were identified in both biopsies (Table 10). This result proves the feasibility to specifically detect *Borrelia* proteins in infected mouse skin at the late stage of the disease by LC-SRM. A maximum of 18 peptides corresponding to 4 proteins (flagellin, VlsE, DbpA and lipoprotein gi|365823350) were detected in the biopsy infected at 259 *fla*/10⁴ *gapdh* (mouse 1). For this biopsy, flagellin was detected with 8 targeted peptides whereas 4 peptides were previously identified by Ge-LC-MS/MS (Table 3). A similar result is observed with the second biopsy infected with *B*. *afzelii*: 9 peptides of flagellin were detected by Ge-LC-SRM whereas 4 peptides were identified by Ge-LC-MS/MS (Table 3). The detection of more peptides thus constitutes an advantage of the targeted LC-SRM method over Ge-LC-MS/MS. By using AQUA® peptides, we estimated the quantity of 3 different flagellin peptides in each biopsy (Table 10).

**Table 10. Targeted detection of bacterial proteins by LC-SRM in mouse skin infected with Borrelia burgdorferi ss N40 or Borrelia afzelii NE4049, intraperitonally (IP) or intradermally (ID), and reactivated with clobetasol 40 days after initial inoculation. The sequences of SRM-monitored peptides are given. N.d. = Not detected, D = Detected, Q = Detected and quantified. Quantifications are bracketed (fmol/mg skin).**

| | | ***B. burgdorferi* ss N40** | | | | ***B. afzelii* NE4049** | | | |
|---|---|---|---|---|---|---|---|---|---|
| | **Infection Mode** | IP | ID | ID | ID | ID | ID | ID | ID |
| | ***Infection rate fla*/*10⁴ gapdh*** | *259* | *43* | *15* | *10* | *304* | *69* | *10* | *6* |
| **Detected proteins** | **Monitored peptides** | **Mouse 1** | **Mouse 2** | **Mouse 3** | **Mouse 4** | **Mouse 5** | **Mouse 6** | **Mouse 7** | **Mouse 8** |
| **DbpA** | DITDEIDAIK (SEQ ID NO: 117) | N.d. | N.d. | N.d. | N.d. | N.d. | N.d. | N.d. | N.d. |
| | GVNFDAFK (SEQ ID NO: 118) | N.d. | N.d. | N.d. | N.d. | N.d. | N.d. | N.d. | N.d. |
| | TTANGIIEIVK (SEQ ID NO: 119) | N.d. | N.d. | N.d. | N.d. | N.d. | N.d. | N.d. | N.d. |
| | TVTDAAEQHPTTTAEGILEIAK (SEQ ID NO: 115) | **D** | N.d. | N.d. | N.d. | N.d. | N.d. | N.d. | N.d. |
| | VSENSFILEAK (SEQ ID NO: | **D** | N.d. | N.d. | N.d. | N.d. | N.d. | N.d. | N.d. |
| | 116) | | | | | | | | |
| **Flagellin** | ANLGAFQNR (SEQ ID NO: 28) | **Q (45)** | **Q (5)** | N.d. | N.d. | **Q (36)** | **Q (4)** | N.d. | N.d. |
| | AINFIQTTEGNLNEVEK (SEQ ID NO: 19) | **Q (94)** | N.d. | N.d. | N.d. | **Q (52)** | N.d. | N.d. | N.d. |
| | INTPASLSGSQASWTLR (SEQ ID NO: 31) | **Q (88)** | **Q (14)** | N.d. | N.d. | **Q (77)** | N.d. | N.d. | N.d. |
| | ASDDAAGMGVSGK (SEQ ID NO: 24) | D | D | N.d. | N.d. | D | N.d. | N.d. | N.d. |
| | ELAVQSGNGTYSDSDR (SEQ ID NO: 30) | N.d. | N.d. | N.d. | N.d. | N.d. | N.d. | N.d. | N.d. |
| | GSIQIEIEQLTDEINR (SEQ ID NO: 33) | N.d. | N.d. | N.d. | N.d. | N.d. | N.d. | N.d. | N.d. |
| | IADQAQYNQMHMLSNK (SEQ ID NO: 27) | **D** | **D** | N.d. | N.d. | **D** | N.d. | N.d. | N.d. |
| | MIINHNTSAINASR (SEQ ID NO: 32) | **D** | **D** | N.d. | N.d. | **D** | N.d. | N.d. | N.d. |
| | NNAINAANLSK (SEQ ID NO: | N.d. | N.d. | N.d. | N.d. | **D** | D | N.d. | N.d. |
| | 18) | | | | | | | | |
| | NNGINAANLSK (SEQ ID NO: 17) | **D** | N.d. | N.d. | N.d. | N.d. | N.d. | N.d. | N.d. |
| | NSTEYAIENLK (SEQ ID NO: 25) | N.d. | N.d. | N.d. | N.d. | **D** | N.d. | N.d. | N.d. |
| | TAEELGMQPAK (SEQ ID NO: 26) | **D** | **D** | N.d. | N.d. | **D** | N.d. | N.d. | N.d. |
| **HP BAPKO**_**0593** | LPLALNLAVSR (SEQ ID NO: 114) | N.d. | N.d. | N.d. | N.d. | **D** | N.d. | N.d. | N.d. |
| **Lipoprotein gi\|365823350** | EFFDWLSK (SEQ ID NO: 110) | **D** | N.d. | N.d. | N.d. | N.d. | N.d. | N.d. | N.d. |
| | GEALSLFFQK (SEQ ID NO: 111) | **D** | N.d. | N.d. | N.d. | N.d. | N.d. | N.d. | N.d. |
| | SLTEIDSGNGIPLVVSDVVK (SEQ ID NO: 109) | N.d. | N.d. | N.d. | N.d. | N.d. | N.d. | N.d. | N.d. |
| | VLTESESNNELK (SEQ ID NO: 112) | **D** | N.d. | N.d. | N.d. | N.d. | N.d. | N.d. | N.d. |
| **Vls13** | AAAAVSSVSGEQILK (SEQ ID NO: 5) | N.d. | N.d. | N.d. | N.d. | N.d. | N.d. | N.d. | N.d. |
| | AVSSVSGEQILK (SEQ ID NO: 6) | N.d. | N.d. | N.d. | N.d. | N.d. | N.d. | N.d. | N.d. |
| | GIVDAAGTAAGK (SEQ ID NO: 2) | **D** | **D** | **D** | N.d. | **D** | N.d. | N.d. | N.d. |
| | GIVDAAGTAAGKK (SEQ ID NO: 1) | **D** | **D** | N.d. | N.d. | N.d. | N.d. | N.d. | N.d. |
| | IGESADNGAAADADSVK (SEQ ID NO: 8) | N.d. | N.d. | N.d. | N.d. | N.d. | N.d. | N.d. | N.d. |
| | VAAALVLR (SEQ ID NO: 4) | **D** | **D** | N.d. | N.d. | **D** | N.d. | N.d. | N.d. |
| **Vls2** | AAEAVSSVSGEQILK (SEQ ID NO: 11) | **D** | **D** | N.d. | N.d. | N.d. | N.d. | N.d. | N.d. |
| | AAEEAIVGATGDGTK (SEQ ID NO: 12) | **D** | **D** | N.d. | N.d. | N.d. | N.d. | N.d. | N.d. |
| | Typing | YES | YES | YES | NO | YES | YES | NO | NO |

Then, we assessed the sensibility of our method by analyzing six additional mouse skin biopsies with lower infection rates (*B. burgdorferi* ss mice 2,3,4 and *B. afzelii* : 6,7,8): 6-69 *fla*/10⁴ *gapdh,* Table 6). These six biopsies were analyzed with the same Ge-LC-SRM approach (50 µg loaded onto the gel, 11 bands). After analysis, we detected *Borrelia* proteins in three biopsies. The two detected proteins were flagellin and VlsE. By using AQUA® peptide, we estimated the quantity of flagellin in two biopsies. Finally, a VIsE peptide (GIVDAAGTAAGK) was detected even at a very low infection rate (15 *fla*/10⁴ *gapdh*)*.* Interestingly, we observed a good correlation between flagellin absolute quantification or total number of detected peptides in each biopsy and the infection rate determined by RT-PCR. Finally, the detection of proteotypic peptides of each *Borrelia* species/strain allowed us bacterial typing when the initial PCR quantification was sufficient (at least 15 *fla*/10⁴ *gapdh*)*.*

### Detection of Borrelia in infected mouse skin after antibiotic treatment

We wanted to determine the persistence of *Borrelia* (live bacteria, DNA and proteins) in the skin after an antibiotic treatment in infected mice (n=29) by culture, PCR and Ge-LC-MS/MS. After one day of treatment, all cultures in BSK-H medium were positive and quantifications were between 0.07 to 0.16 *fla*/10⁴ *gapdh,* but no protein was identified by Ge-LC-MS/MS. From the second day of treatment, all cultures, PCRs and protein detection were negatives (data not shown).

### Discussion

The purpose of this study was first to demonstrate the organotropism of *Borrelia* for the skin, then to use the skin as an organ of persistent bacterial infection to identify markers of active infection for late Lyme borreliosis. The 40 days was chosen to mimic a chronic phase of the disease on this C3H/HeN mouse model (Barthold et al. 1990 **([22])**). Interestingly, whatever the route of inoculation, intradermal or intraperitoneal, *Borreliae* were found in the skin, demonstrating that the skin is an essential organ for the development and persistence of this disease. We found that these *Borreliae* were alive, infectious to the ticks in absence of reactivation and able to multiply (after corticosteroid application). Multiplication rate was greater when *Borrelia* was inoculated intraperitoneally than intradermally. The biodiversity of *B. burgdorferi* varies according to its environment: it is greater in tick populations, less diverse in the skin, and even less diverse in disseminated sites (Brisson et al. 2011 **([23)**). Intraperitoneal inoculation, avoiding cutaneous immunity, may preserve this diversity, and more genotypes could survive in the mouse than via intradermally inoculation. As for all arthropod borne diseases (ABDs), it is likely that some pathogens remain in the skin to insure a better transmission to uninfected arthropod, tick or insect. It has been demonstrated in other diseases where pathogens disseminate to deep organs that, some of them remain in the skin: malaria (Ménard et al. 2013 **([24])**), trypanosomiasis (Caljon et al. 2016 **([25])**), anaplasmosis (Granquist et al. 2010 **([26])**) as a few exemples. During an infectious blood meal, the tick biting pieces dilacerate the epidermis and reaches the dermis, where *Borrelia* is inoculated. This induces a local immune response constituted by the secretion of chemokines, cytokines and antimicrobial peptides (AMPs) (Jones et al. 2008 **([27])**). This immune process is actively involved in bacterial clearance (Müllegger et al. 2007 **([28])**), however some bacteria can escape and remain in different tissues as shown here with *Borrelia.*
We then investigated the process of *Borrelia* acquisition by uninfected tick and the potential immunosuppressive effect of tick saliva on this process. During the early transmission of *Borrelia* to the vertebrate host, tick saliva is known to have immunomodulatory properties and assist the bacteria to escape host immune response. It contains very diverse molecules with anti-hemostatic, antiinflammatory and immunomodulatory properties to facilitate the uptake of the blood meal that is essential for their survival (Francischetti et al. 2009 **([29])**; Kazimírová and Stibrániová **([21])**; Titus et al. 2006 **([30])**). Tick saliva is critical for effective pathogen transmission to the vertebrate host. Its role in the early transmission of pathogen is well studied, but studies on its role in the acquisition of pathogen by a naïve host in disseminated infections are very scarce (Shih et al. 1995 **([31])**).
We then analyzed the process of *Borrelia* transmission from the infected vertebrate host to the naive ticks to confirm the importance of the skin in this ABDs. To this end, we put uninfected ticks on infected mice and collected the ticks at different time points. Ticks were then tested by PCR to check the presence of *Borrelia.* The time necessary for a tick to acquire the bacteria was very quick, since already 5 hours after the beginning of the blood meal, *Borrelia* was detected in the ticks. It correlates with another study which showed that *I. scapularis,* the american tick, acquires *Borrelia* 8 hours after feeding on mice (Shih et al. **([31])**). Tick saliva is well-known for its immunosuppressive effect on the skin immunity, especially during *Borrelia* transmission to uninfected vertebrate host (Kazimírová and Stibrániová **([21])**). We were expecting to get a similar process of immunosuppression by the tick which feeds on infected host, triggering a local bacterial multiplication that would facilitate pathogen acquisition. If an immunosuppression occurs, we were not able to detect it by PCR since no significant bacterial multiplication was measured in the skin. Although it could take place, the PCR was not sensitive enough to detect the bacteria in the skin.

Then, we used a dermocorticoid to boost locally the bacteria in the skin to be able to detect them by PCR and then to perform proteomics studies to demonstrate their viability. We have previously shown that proteomics of *Borrelia* in the skin is successful if the bacteria multiply significantly. This experiment was performed during the early transmission of bacteria to mice at the time point where *Borrelia* multiplies in the mouse skin, at day 7 after intradermal multiplication (Schnell et al. 2015 **([15])**). We then used cortisone to boost locally *Borrelia* to detect markers of late infection in the skin and we succeeded. Flagellin was detected but also VlsE. VIsE is a well-known protein of *Borrelia* appearing in disseminated infection (Liang et al. 2000 **([32])**; Jacek et al. 2016 **([33])**). This protein is used in ELISA for diagnostic of late disseminated infection in Lyme disease (Aguero-Rosenfeld et al. 2005 **([8])**). Then, to mimic as well as possible the transmission of *Borrelia,* we used ticks infected with *B. afzelii* NE4049. With this protocol of transmission and after dermocorticoid reactivation, we got a large panel of bacterial proteins like flagellin and VlsE, but also L-lactate dehydrogenase, Neutrophil activating protein (Nap), enolase, oligopeptide ABC transporter, Elongation factor Tu. Elongation factor Tu is a highly immunogenic protein during Lyme borreliosis which primarily localizes in the protoplasmic cylinder of spirochetes and is not on the surface of *B. burgdorferi* (Carrasco et al. 2015 **([34])**). Nap is one of the main bacterial product involved in pathogenesis of Lyme arthritis, especially in early stages of Lyme arthritis. It orchestrates the recruitment of inflammatory cells into the joint cavity (Codolo et al. 2013 **([35])**). Its role in late stages of skin borreliosis is unknown and its presence was unexpected. Peptide ABC transporters may play a major role in nutrition for the organism and in bacterial virulence (Lin et al. 2001 **([36])**; Garmory and Tiball 2004 **([37])**). Several identified proteins like GroEL, oligopeptide ABC transporter, enolase, GAPDH are known to be expressed in virulence vesicles in *Borrelia* (Toledo et al. 2012 **([38])**). They are all expressed in the process of this artificial multiplication.
Among the three most pathogenic species of *Borrelia* infecting humans, *B. afzelii* NE4049, a strain that circulates very well in Nature (Tonetti et al. 2015 **([17])**), was found to persist and multiply the best in the skin. Rodents are the best reservoir for this species, while *B. garinii* has a bird reservoir and *B. burgdorferi* ss is ubiquitous (Humair and Gern 2000 **([39])**). This could explain the efficient persistence and multiplication of *B. afzelii* in mouse skin compared to *B. garinii.*
Since these bacterial proteins detected after reactivation are present in disseminated infections, we propose to use them as markers of late infections for Lyme borreliosis in patients. We have shown that this approach is feasible in early diagnostic of Lyme borreliosis (Schnell et al. 2015 **([15])**). We are validating this approach in more patients and we are comparing its efficiency and sensitivity to PCR detection and *Borrelia* culture (personal communication). The skin remains positive for months in mouse model (Barthold et al. 1993 **([40])**) but also in dogs (Krupka and Straubinger 2010 **([41])**). Interestingly, the overall skin of these animals is positive in absence of antibiotic treatment. In human, the overall positivity of the skin is not known. However, tick bite remains visible for several months in some patients and skin biopsies could be taken at the site of the tick bite to perform the diagnostic. Alternatively, proteomics might be used on other biological fluids such as synovial fluid or cerebrospinal fluid. In addition, we clearly demonstrate that after only one day of ceftriaxone treatment in mouse, no live bacteria, DNA and proteins are detected. Diagnostics relying on proteomics could help clinicians to distinguish late stage borreliosis efficiently treated with antibiotics, from persistent infections or reinfections.
Late lyme disease is a real problem in humans (Wormser et al. 2006 **([42])**; Stanek et al. 2012 **([43])**). Most of the time, it is linked to inflammatory process persisting in patients, while the bacteria have been killed by efficient antibiotic treatment. Alternatively, it could be either due to an inefficient antibiotic treatment and the persistence of resistant *Borrelia* in tissue, however this aspect of Lyme borreliosis is controversial. The proteomics, because it detects proteins, then metabolically active organisms, could be used in these patients to check for the real persistence of *Borrelia* in their tissues. Such an approach deserves further investigation to succeed in new diagnostic tools for late Lyme borreliosis in humans.

### Listes des références

1. Radolf, J.D., Caimano, M.J., Stevenson, B., and Hu, L.T. (2012). Of ticks, mice and men: understanding the dual-host lifestyle of Lyme disease spirochaetes. Nature reviews Microbiology 10, 87-99.
2. Piesman, J., Schneider, B.S., and Zeidner, N.S. (2001). Use of quantitative PCR to measure density of Borrelia burgdorferi in the midgut and salivary glands of feeding tick vectors. Journal of clinical microbiology 39, 4145-4148.
3. Kern, A, Collin, E, Barthel, C, et al. (2011). Tick saliva represses innate immunity and cutaneous inflammation in a murine model of lyme disease. Vector Borne Zoonotic Dis 11: 1343-50.
4. Shih, C.M., Pollack, R.J., Telford, S.R., 3rd, and Spielman, A. (1992). Delayed dissemination of Lyme disease spirochetes from the site of deposition in the skin of mice. The Journal of infectious diseases 166, 827-831.
5. Stanek, G, Wormser, GP, Gray, J, et al. (2012). Lyme borreliosis. Lancet 379: 461-73.
6. Moriarty, T.J., Norman, M.U., Colarusso, P., Bankhead, T., Kubes, P., and Chaconas, G. (2008). Real-time high resolution 3D imaging of the lyme disease spirochete adhering to and escaping from the vasculature of a living host. PLoS pathogens 4, e1000090.
7. Leeflang, MMG, Ang, CW, Berkhout, J, et al. (2016). The diagnostic accuracy of serological tests for Lyme borreliosis in Europe: a systematic review and meta-analysis. BMC Infect Dis 16: 140.
8. Aguero-Rosenfeld, M, Wang, G, Schwartz, I, et al. (2005). Diagnosis of lyme borreliosis. Clin Microbiol Rev 18: 484-509.
9. Domon, B. and Gallien, S. (2015). Recent advances in targeted proteomics for clinical applications. Proteomics Clin Appl. 3-4: 423-31.
10. Kruh-Garcia, N., Wolfe, L., Chaisson, L., Worodria, W., Nahid, P., Schorey, J., et al. (2014). Detection of Mycobacterium tuberculosis peptides in the exosomes of patients with active and latent M. tuberculosis infection using MRM-MS. PLoS One. 31: e103811.
11. Percy, A., Yang, J., Chambers, A., Mohammed, Y., Miliotis, T., Borchers, C. (2016). Protocol for Standardizing High-to-Moderate Abundance Protein Biomarker Assessments Through an MRM-with-Standard-Peptides Quantitative Approach. Adv Exp Med Biol. 919: 515-530.
12. Huttenhain, R., Soste, M., Selevsek, N., Rost, H., Sethi, A., Carapito, C., et al. (2012). Reproducible Quantification of Cancer-Associated Proteins in Body Fluids Using Targeted Proteomics. Sci Transl Med 4: 142ra94-142ra94.
13. Kennedy, J.J., Abbatiello, S.E., Kim, K., et al. (2014). Demonstrating the feasibility of large-scale development of standardized assays to quantify human proteins. Nat Methods 11: 149-55.
14. Percy, A.J., Chambers, A.G., Yang, J., et al. (2013). Multiplexed MRM-based quantitation of candidate cancer biomarker proteins in undepleted and non-enriched human plasma. Proteomics 13: 2202-15.
15. Schnell, G., Boeuf, A., Westermann, B., et al. (2015). Discovery and targeted proteomics on cutaneous biopsies: a promising work toward an early diagnosis of Lyme disease. Mol Cell proteomics 14: 1254-64.
16. Mbow, ML, Christe, M, Rutti, B, et al. (1994). Absence of acquired resistance to nymphal Ixodes ricinus ticks in BALB/c mice developing cutaneous reactions. J Parasitol 80: 81-7.
17. Tonetti, N, Voordouw, M, Durand, J, et al. (2015). Genetic variation in transmission success of the Lyme borreliosis pathogen Borrelia afzelii. Tick Borne Dis 6: 334-43.
18. Jaulhac, B., Heller, R., Limbach, F.X., Hansmann, Y., Lipsker, D., Monteil, H., Sibilia, J., and Piemont, Y. (2000). Direct molecular typing of Borrelia burgdorferi sensu lato species in synovial samples from patients with lyme arthritis. Journal of clinical microbiology 38, 1895-1900.
19. Muller, L., Fornecker, L., Van Dorsselaer, A., Cianférani, S., Carapito, C. (2016). Benchmarking sample preparation/digestion protocols reveals tube-gel being a fast and repeatable method for quantitative proteomics. Proteomics. 16: 2953-2961.
20. Elias, JE, Gygi, SP (2007). Target-decoy search strategy for increased confidence in large-scale protein identifications by mass spectrometry. Nat Methods 4: 207-14.
21. Kazimírová, M, Stibrániová, I (2013). Tick salivary compounds: their role in modulation of host defences and pathogen transmission. Front Cell Infect Microbiol 3: 1-17.
22. Barthold, S.W., Beck, D.S., Hansen, G.M., Terwilliger, G.A., and Moody, K.D. (1990). Lyme borreliosis in selected strains and ages of laboratory mice. The Journal of infectious diseases 162, 133-138.
23. Brisson, D, Baxamusa, N, Schwartz, I, et al. (2011). Biodiversity of borrelia burgdorferi strains in tissues of lyme disease patients. PLoS One 6.
24. Ménard, R, Tavares, J, Cockburn, I, et al. (2013). Looking under the skin: the first steps in malarial infection and immunity. Nat Rev Microbiol 11: 701-12.
25. Caljon, G, Van Reet, N, De Trez, C, et al. (2016). The Dermis as a Delivery Site of Trypanosoma brucei for Tsetse Flies. PLoS Pathog 12: e1005.
26. Granquist, E, Aleksandersen, M, Bergström, K, et al. (2010). A morphological and molecular study of Anaplasma phagocytophilum transmission events at the time of Ixodes ricinus tick bite. Acta Vet Scand 17: 43.
27. Jones, K.L., Muellegger, R.R., Means, T.K., Lee, M., Glickstein, L.J., Damle, N., Sikand, V.K., Luster, A.D., and Steere, A.C. (2008). Higher mRNA levels of chemokines and cytokines associated with macrophage activation in erythema migrans skin lesions in patients from the United States than in patients from Austria with Lyme borreliosis. Clin Infect Dis 46, 85-92.
28. Müllegger, R.R., Means, T.K., Shin, J.J., Lee, M., Jones, K.L., Glickstein, L.J., Luster, A.D., and Steere, A.C. (2007). Chemokine signatures in the skin disorders of Lyme borreliosis in Europe: predominance of CXCL9 and CXCL10 in erythema migrans and acrodermatitis and CXCL13 in lymphocytoma. Infection and immunity 75, 4621-4628.
29. Francischetti, I.M., Sa-Nunes, A., Mans, B.J., Santos, I.M., and Ribeiro, J.M. (2009). The role of saliva in tick feeding. Frontiers in bioscience 14, 2051-2088.
30. Titus, R.G., Bishop, J.V., and Mejia, J.S. (2006). The immunomodulatory factors of arthropod saliva and the potential for these factors to serve as vaccine targets to prevent pathogen transmission. Parasite immunology 28, 131-141.
31. Shih, C, Liu, L, Spielman, A (1995). Differential spirochetal infectivities to vector ticks of mice chronically infected by the agent of Lyme disease. J Clin Microbiol. 1995 Dec;33(12):3164-8. J Microbiol 33: 3164-8.
32. Liang, F, Aberer, E, Cinco, M, et al. (2000). Antigenic conservation of an immunodominant invariable region of the VIsE lipoprotein among European pathogenic genospecies of Borrelia burgdorferi SL. J Infect Dis 182: 1455662.
33. Jacek, E, Tang, K, L, K, et al. (2016). Epitope-Specific Evolution of Human B Cell Responses to Borrelia burgdorferi VIsE Protein from Early to Late Stages of Lyme Disease. J Immunol 196: 1036-43.
34. Carrasco, SE, Yang, Y, Troxell, B, et al. (2015). Borrelia burgdorferi elongation factor EF-Tu is an immunogenic protein during Lyme borreliosis. Emerg Microbes Infect 4: e54.
35. Codolo, G, Bossi, F, Durigutto, P, et al. (2013). Orchestration of inflammation and adaptive immunity in borrelia burgdorferi-induced arthritis by neutrophil-activating protein A. Arthritis Rheum 65: 1232-42.
36. Lin, B, Short, SA, Eskildsen, M, et al. (2001). Functional testing of putative oligopeptide permease (Opp) proteins of Borrelia burgdorferi: A complementation model in opp- Escherichia coli. Biochim Biophys Acta - Mol Cell Res 1499: 222-31.
37. Garmory H, Titball R. ATP-binding cassette transporters are targets for the development of antibacterial vaccines and therapies. Infect Immun. 2004;72: 6757-63.
38. Toledo, A, Coleman, J, Kuhlow, C, et al. (2012). The enolase of Borrelia burgdorferi is a plasminogen receptor released in outer membrane vesicles. Infect Immun 80: 359-68.
39. Humair, P, Gern, L (2000). The wild hidden face of Lyme borreliosis in Europe. Microbes Infect 2: 915-22.
40. Barthold, S, de Souza, M, Janotka, J, et al. (1993). Chronic Lyme borreliosis in the laboratory mouse. Am J Pathol 143: 959-71.
41. Krupka, I, Straubinger, R (2010). Lyme borreliosis in dogs and cats: background, diagnosis, treatment and prevention of infections with Borrelia burgdorferi sensu stricto. Vet Clin North Am Small Anim Pr 40: 1103-19.
42. Wormser, GP, Dattwyler, RJ, Shapiro, ED, et al. (2006). The clinical assessment, treatment, and prevention of lyme disease, human granulocytic anaplasmosis, and babesiosis: clinical practice guidelines by the Infectious Diseases Society of America. Clin Infect Dis 43: 1089-134.
43. Stanek, G, Wormser, GP, Gray, J, et al. (2012). Lyme borreliosis. Lancet 379: 461-73.

## Claims

1. An *ex vivo* method of detecting a *Borrelia burgdorferi* sensu lato active infection in a skin sample of a subject, said skin sample being treated with an immunosuppressive dermocorticoid before sampling, comprising the step of detecting, in said skin sample, at least one protein of *Borrelia burgdorferi* sensu lato chosen in the group comprising VIsE, flagellin, GAPDH, flagellar filament outer layer protein, L-lactate dehydrogenase, enolase, oligopeptide ABC transporter periplasmic oligopeptide-binding protein, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog thereof, and DbpA.

2. The method of claim 1, wherein the group comprises VIsE, flagellin and GAPDH.

3. The method of claim 1, wherein the group comprises at least one protein chosen in the group comprising VlsE, flagellin and GAPDH, and at least one protein chosen in the group comprising flagellar filament outer layer protein, L-lactate dehydrogenase, enolase, oligopeptide ABC transporter periplasmic oligopeptide-binding protein, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog thereof, and DbpA.

4. The method of claim 1, wherein the group comprises VIsE, flagellin, GAPDH, flagellar filament outer layer protein, L-lactate dehydrogenase, enolase, oligopeptide ABC transporter periplasmic oligopeptide-binding protein, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog thereof, and DbpA.

5. The method of anyone of the preceding claims, wherein the detection is a quantification of said at least one polypeptide.

6. The method of anyone of the preceding claims, wherein the detecting step of at least one protein is a detection of at least one peptide corresponding to said at least one protein.

7. The method of claim 6, wherein:
- VlsE detection is a detection of at least one peptide selected in the group comprising:
GIVDAAGTAAGKK (SEQ ID NO: 1),
GIVDAAGTAAGK (SEQ ID NO: 2),
DKVAAALVLR (SEQ ID NO: 3),
VAAALVLR (SEQ ID NO: 4),
AAAAVSSVSGEQILK (SEQ ID NO: 5),
AVSSVSGEQILK (SEQ ID NO: 6),
FSVTNANDANVK (SEQ ID NO: 7),
IGESADNGAAADADSVK (SEQ ID NO: 8),
AAGAVSAVSGEQILK (SEQ ID NO: 9),
FSAADGDGANVK (SEQ ID NO: 10),
AAEAVSSVSGEQILK (SEQ ID NO: 11),
AAEEAIVGATGDGTK (SEQ ID NO: 12),
KDDQIAAAIALR (SEQ ID NO: 13),
DDQIAAAIALR (SEQ ID NO: 14),
AIVDAAGK (SEQ ID NO: 15), and
NPIAAAIGNGNGAGFVDADMR (SEQ ID NO: 16);
- flagellin detection is a detection of at least one peptide selected in the group comprising:
NNGINAANLSK (SEQ ID NO: 17),
NNAINAANLSK (SEQ ID NO: 18),
AINFIQTTEGNLNEVEK (SEQ ID NO: 19),
ASYAQK (SEQ ID NO: 20),
INAQIR (SEQ ID NO: 21),
DSTEYAIENLK (SEQ ID NO: 22),
LESIKDSTEYAIENLK (SEQ ID NO: 23),
ASDDAAGMGVSGK (SEQ ID NO: 24),
NSTEYAIENLK (SEQ ID NO: 25),
TAEELGMQPAK (SEQ ID NO: 26),
IADQAQYNQMHMLSNK (SEQ ID NO: 27),
ANLGAFQNR (SEQ ID NO: 28),
MKELAVQSGNGTYSDSDR (SEQ ID NO: 29),
ELAVQSGNGTYSDSDR (SEQ ID NO: 30),
INTPASLSGSQASWTLR (SEQ ID NO: 31),
MIINHNTSAINASR (SEQ ID NO: 32) and
GSIQIEIEQLTDEINR (SEQ ID NO: 33);
- GAPDH detection is a detection of at least one peptide selected in the group comprising:
IIAERDPK (SEQ ID NO: 34),
AVGLVLPELK (SEQ ID NO: 35),
VPVPTGSIVDLTVQLK (SEQ ID NO: 36),
GIDIVAINDLTDPK (SEQ ID NO: 37),
GGYLDHVNHAGAK (SEQ ID NO: 38),
VILTVPAKDEIK (SEQ ID NO: 39),
TIVLGVNDHDINSDLK (SEQ ID NO: 40),
AAALSIIPTSTGAAK (SEQ ID NO: 41),
AVSNASCTTNCLAPLAK (SEQ ID NO: 42),
ILDLPHSDLRR (SEQ ID NO: 43),
ILDLPHSDLR (SEQ ID NO: 44),
VLHESFGIEQGLMTTVHAYTNDQR (SEQ ID NO: 45),
VLSWYDNEFGYSTR (SEQ ID NO: 46) and
LAINGFGR (SEQ ID NO: 47);
- L-lactate dehydrogenase detection is a detection of at least one peptide selected in the group comprising:
DCVNADIVVITAGLNQKPGETR (SEQ ID NO: 48),
GATYYAIGLGIK (SEQ ID NO: 49),
ITEIELDEIHKK (SEQ ID NO: 50),
VIGTGTILDTSR (SEQ ID NO: 51),
IAMKPLSEYLSEGK (SEQ ID NO: 52) and
NIVNAIIGDQNIILPISSYINGQYGGLIK (SEQ ID NO: 53);
- flagellar filament outer layer protein detection is a detection of at least one peptide selected in the group comprising:
IIKDDVPNYPLASSK (SEQ ID NO: 54),
ISMPEGSFQNFIEK (SEQ ID NO: 55),
YAGDTILGVR (SEQ ID NO: 56),
VYETSGAESLR (SEQ ID NO: 57),
NSVVAPALVK (SEQ ID NO: 58),
AEPGELVLDFAELAR (SEQ ID NO: 59) and
RAEPGELVLDFAELAR (SEQ ID NO: 60);
- enolase detection is a detection of at least one peptide selected in the group comprising:
GYATSVGDEGGFAPNLK (SEQ ID NO: 61),
SAVPSGASTGINEAVELR (SEQ ID NO: 62),
VNQIGTLTETFEAVEMAK (SEQ ID NO: 63),
SGETEDTTIADLVVALGTGQIK (SEQ ID NO: 64),
VYQYLGAYK (SEQ ID NO: 65) and
IQLVGDDLFVTNTSFLK (SEQ ID NO: 66);
- oligopeptide ABC transporter, periplasmic oligopeptide-binding protein detection is a detection of at least one peptide selected in the group comprising:
ITADAIR (SEQ ID NO: 67),
ALTLAIDR (SEQ ID NO: 68),
DFPIAPIYIYGNSYLFR (SEQ ID NO: 69),
KAEEIIIEK (SEQ ID NO: 70),
FDLSQLK (SEQ ID NO: 71),
YGQSWTNPENIVTSGPFK (SEQ ID NO: 72),
SDYYSSAVNAIYFYAFNTHIKPLDNVK (SEQ ID NO: 73),
NLELFNPEIAK (SEQ ID NO: 74),
TLEITLESPKPYFIDMLVHQSFIPIPIHIAEK (SEQ ID NO: 75),
GLITGDPNTGGNKPGLAK (SEQ ID NO: 76),
ETGSNYSEMVK (SEQ ID NO: 77) and
SWDISPDGTVYTFTLR (SEQ ID NO: 78);
- neutrophil activating protein detection is a detection of at least one peptide selected in the group comprising:
IIDIIAER (SEQ ID NO: 79),
KDDLEEIHSK (SEQ ID NO: 80),
TQDLYEYIEK (SEQ ID NO: 81),
LIDTACDYGTANLIDDIMSDLEK (SEQ ID NO: 82) and
LQELLASLHIFYSNLR (SEQ ID NO: 83);
- 2,3-biphosphoglycerate-dependent phosphoglycerate mutase detection is a detection of at least one peptide selected in the group comprising:
ANDTLNIILK (SEQ ID NO: 84),
LNIPTGIPLVYELDK (SEQ ID NO: 85),
GVDEAIEAGLLLK (SEQ ID NO: 86),
HYGALQGLNK (SEQ ID NO: 87) and
LNIPTGIPLVYELDKDLNPIK (SEQ ID NO: 88);
- elongation factor Tu detection is a detection of at least one peptide selected in the group comprising:
DIDKPFLLAVEDVFSISGR (SEQ ID NO: 89),
TTDVTGVVALEGK (SEQ ID NO: 90),
GSAFGAMSNPEDPEATK (SEQ ID NO: 91),
YGFSANTPIIK (SEQ ID NO: 92),
VGQEVEIVGIK (SEQ ID NO: 93),
GQVLSAPGTITPHKK (SEQ ID NO: 94),
ALKYEDIDNAPEEK (SEQ ID NO: 95),
TTLTAAISIYCSK (SEQ ID NO: 96),
ILEQGQAGDNVGLLLR (SEQ ID NO: 97),
YEDIDNAPEEK (SEQ ID NO: 98) and
HIEYETANR (SEQ ID NO: 99);
- GroEL detection is a detection of at least one peptide selected in the group comprising:
ELLPVLEK (SEQ ID NO: 100),
SALQNAASIAGLLLTTECAITDIKEEK (SEQ ID NO: 101),
QIISNAGFEGSIYIHQIK (SEQ ID NO: 102),
AAVEEGVVPGGGSTLIEVAMYLDTIDTSK (SEQ ID NO: 103),
VDKDNTTIINTGNK (SEQ ID NO: 104),
NVSSGINPIGIK (SEQ ID NO: 105) and
WVNMIESGIIDPAK (SEQ ID NO: 106);
- Lipoprotein gi|365823350 or gi|256055301 or any *Borrelia* species homolog thereof detection is a detection of at least one peptide selected in the group comprising:
EISLTPEEAEKLESLK (SEQ ID NO: 107),
SLTEIYSGNGIPLVVSDVVK (SEQ ID NO: 108),
SLTEIDSGNGIPLVVSDVVK (SEQ ID NO: 109),
EFFDWLSK (SEQ ID NO: 110),
GEALSLFFQK (SEQ ID NO: 111) and
VLTESESNNELK (SEQ ID NO: 112);
- Hypothetical protein BbuN40_0563 or any *Borrelia* species homolog thereof detection is a detection of at least one peptide selected in the group comprising:
LPLALNIAVFR (SEQ ID NO: 113) and
LPLALNLAVSR (SEQ ID NO: 114);
- DbpA detection is a detection of at least one peptide selected in the group comprising:
TVTDAAEQHPTTTAEGILEIAK (SEQ ID NO: 115),
VSENSFILEAK (SEQ ID NO: 116),
DITDEIDAIK (SEQ ID NO: 117),
GVNFDAFK (SEQ ID NO: 118) and
TTANGIIEIVK (SEQ ID NO: 119).

8. The method of anyone of the preceding claims, wherein the immunosuppressive dermocorticoid is chosen in the group comprising clobetasol, béthaméthasone and hydrocortisone.

9. The method of anyone of the preceding claims, for the diagnosis of late disseminated Lyme borreliosis in humans or animals.

10. The method of anyone of the preceding claim, wherein the detection step comprises a mass spectrometry analysis.

11. Set of polypeptides of *Borrelia burgdorferi* sensu lato comprising :
- VIsE protein, flagellin protein and GAPDH protein, and eventually,
- at least one polypeptide chosen in the group comprising flagellar filament outer layer protein, L-lactate dehydrogenase, enolase, oligopeptide ABC transporter periplasmic oligopeptide-binding protein, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog thereof and DbpA.

12. Set of polypeptides comprising VIsE protein, flagellin protein and GAPDH protein, and at least one polypeptide chosen in the group comprising flagellar filament outer layer protein, L-lactate dehydrogenase, enolase, oligopeptide ABC transporter periplasmic oligopeptide-binding, neutrophil activating protein, 2,3-biphosphoglycerate-dependent phosphoglycerate mutase, elongation factor Tu, GroEL, Lipoprotein gi|256055301 or gi|365823350 or any *Borrelia* species homolog thereof, Hypothetical protein BbuN40_0563 or any *Borrelia* species homolog and DbpA.

13. Set of polypeptides according to claim 11 or 12, wherein:
- VIsE protein is at least one peptide selected in the group comprising:
GIVDAAGTAAGKK (SEQ ID NO: 1),
GIVDAAGTAAGK (SEQ ID NO: 2),
DKVAAALVLR (SEQ ID NO: 3),
VAAALVLR (SEQ ID NO: 4),
AAAAVSSVSGEQILK (SEQ ID NO: 5),
AVSSVSGEQILK (SEQ ID NO: 6),
FSVTNANDANVK (SEQ ID NO: 7),
IGESADNGAAADADSVK (SEQ ID NO: 8),
AAGAVSAVSGEQILK (SEQ ID NO: 9),
FSAADGDGANVK (SEQ ID NO: 10),
AAEAVSSVSGEQILK (SEQ ID NO: 11),
AAEEAIVGATGDGTK (SEQ ID NO: 12),
KDDQIAAAIALR (SEQ ID NO: 13),
DDQIAAAIALR (SEQ ID NO: 14),
AIVDAAGK (SEQ ID NO: 15) and
NPIAAAIGNGNGAGFVDADMR (SEQ ID NO: 16);
- flagellin protein is at least one peptide selected in the group comprising:
NNGINAANLSK (SEQ ID NO: 17),
NNAINAANLSK (SEQ ID NO: 18),
AINFIQTTEGNLNEVEK (SEQ ID NO: 19),
ASYAQK (SEQ ID NO: 20),
INAQIR (SEQ ID NO: 21),
DSTEYAIENLK (SEQ ID NO: 22),
LESIKDSTEYAIENLK (SEQ ID NO: 23),
ASDDAAGMGVSGK (SEQ ID NO: 24),
NSTEYAIENLK (SEQ ID NO: 25),
TAEELGMQPAK (SEQ ID NO: 26),
IADQAQYNQMHMLSNK (SEQ ID NO: 27),
ANLGAFQNR (SEQ ID NO: 28),
MKELAVQSGNGTYSDSDR (SEQ ID NO: 29),
ELAVQSGNGTYSDSDR (SEQ ID NO: 30),
INTPASLSGSQASWTLR (SEQ ID NO: 31),
MIINHNTSAINASR (SEQ ID NO: 32) and
GSIQIEIEQLTDEINR (SEQ ID NO: 33);
- GAPDH protein is at least one peptide selected in the group comprising:
IIAERDPK (SEQ ID NO: 34),
AVGLVLPELK (SEQ ID NO: 35),
VPVPTGSIVDLTVQLK (SEQ ID NO: 36),
GIDIVAINDLTDPK (SEQ ID NO: 37),
GGYLDHVNHAGAK (SEQ ID NO: 38),
VILTVPAKDEIK (SEQ ID NO: 39),
TIVLGVNDHDINSDLK (SEQ ID NO: 40),
AAALSIIPTSTGAAK (SEQ ID NO: 41),
AVSNASCTTNCLAPLAK (SEQ ID NO: 42),
ILDLPHSDLRR (SEQ ID NO: 43),
ILDLPHSDLR (SEQ ID NO: 44),
VLHESFGIEQGLMTTVHAYTNDQR (SEQ ID NO: 45),
VLSWYDNEFGYSTR (SEQ ID NO: 46) and
LAINGFGR (SEQ ID NO: 47);
- DbpA protein is at least one peptide selected in the group comprising:
TVTDAAEQHPTTTAEGILEIAK (SEQ ID NO: 115),
VSENSFILEAK (SEQ ID NO: 116),
DITDEIDAIK (SEQ ID NO: 117),
GVNFDAFK (SEQ ID NO: 118) and
TTANGIIEIVK (SEQ ID NO: 119);
- L-lactate dehydrogenase protein is at least one peptide selected in the group comprising:
DCVNADIVVITAGLNQKPGETR (SEQ ID NO: 48),
GATYYAIGLGIK (SEQ ID NO: 49),
ITEIELDEIHKK (SEQ ID NO: 50),
VIGTGTILDTSR (SEQ ID NO: 51),
IAMKPLSEYLSEGK (SEQ ID NO: 52) and
NIVNAIIGDQNIILPISSYINGQYGGLIK (SEQ ID NO: 53);
- flagellar filament outer layer protein is at least one peptide selected in the group comprising:
IIKDDVPNYPLASSK (SEQ ID NO: 54),
ISMPEGSFQNFIEK (SEQ ID NO: 55),
YAGDTILGVR (SEQ ID NO: 56),
VYETSGAESLR (SEQ ID NO: 57),
NSVVAPALVK (SEQ ID NO: 58),
AEPGELVLDFAELAR (SEQ ID NO: 59) and
RAEPGELVLDFAELAR (SEQ ID NO: 60);
- enolase protein is at least one peptide selected in the group comprising:
GYATSVGDEGGFAPNLK (SEQ ID NO: 61),
SAVPSGASTGINEAVELR (SEQ ID NO: 62),
VNQIGTLTETFEAVEMAK (SEQ ID NO: 63),
SGETEDTTIADLVVALGTGQIK (SEQ ID NO: 64),
VYQYLGAYK (SEQ ID NO: 65) and
IQLVGDDLFVTNTSFLK (SEQ ID NO: 66);
- oligopeptide ABC transporter periplasmic oligopeptide-binding protein is at least one peptide selected in the group comprising:
ITADAIR (SEQ ID NO: 67),
ALTLAIDR (SEQ ID NO: 68),
DFPIAPIYIYGNSYLFR (SEQ ID NO: 69),
KAEEIIIEK (SEQ ID NO: 70),
FDLSQLK (SEQ ID NO: 71),
YGQSWTNPENIVTSGPFK (SEQ ID NO: 72),
SDYYSSAVNAIYFYAFNTHIKPLDNVK (SEQ ID NO: 73),
NLELFNPEIAK (SEQ ID NO: 74),
TLEITLESPKPYFIDMLVHQSFIPIPIHIAEK (SEQ ID NO: 75),
GLITGDPNTGGNKPGLAK (SEQ ID NO: 76),
ETGSNYSEMVK (SEQ ID NO: 77) and
SWDISPDGTVYTFTLR (SEQ ID NO: 78);
- neutrophil activating protein protein is at least one peptide selected in the group comprising:
IIDIIAER (SEQ ID NO: 79),
KDDLEEIHSK (SEQ ID NO: 80),
TQDLYEYIEK (SEQ ID NO: 81),
LIDTACDYGTANLIDDIMSDLEK (SEQ ID NO: 82) and
LQELLASLHIFYSNLR (SEQ ID NO: 83);
- 2,3-biphosphoglycerate-dependent phosphoglycerate mutase protein is at least one peptide selected in the group comprising:
ANDTLNIILK (SEQ ID NO: 84),
LNIPTGIPLVYELDK (SEQ ID NO: 85),
GVDEAIEAGLLLK (SEQ ID NO: 86),
HYGALQGLNK (SEQ ID NO: 87) and
LNIPTGIPLVYELDKDLNPIK (SEQ ID NO: 88);
- elongation factor Tu protein is at least one peptide selected in the group comprising:
DIDKPFLLAVEDVFSISGR (SEQ ID NO: 89),
TTDVTGVVALEGK (SEQ ID NO: 90),
GSAFGAMSNPEDPEATK (SEQ ID NO: 91),
YGFSANTPIIK (SEQ ID NO: 92),
VGQEVEIVGIK (SEQ ID NO: 93),
GQVLSAPGTITPHKK (SEQ ID NO: 94),
ALKYEDIDNAPEEK (SEQ ID NO: 95),
TTLTAAISIYCSK (SEQ ID NO: 96),
ILEQGQAGDNVGLLLR (SEQ ID NO: 97),
YEDIDNAPEEK (SEQ ID NO: 98) and
HIEYETANR (SEQ ID NO: 99);
- GroEL is at least one peptide selected in the group comprising:
ELLPVLEK (SEQ ID NO: 100),
SALQNAASIAGLLLTTECAITDIKEEK (SEQ ID NO: 101),
QIISNAGFEGSIYIHQIK (SEQ ID NO: 102),
AAVEEGVVPGGGSTLIEVAMYLDTIDTSK (SEQ ID NO: 103),
VDKDNTTIINTGNK (SEQ ID NO: 104),
NVSSGINPIGIK (SEQ ID NO: 105) and
WVNMIESGIIDPAK (SEQ ID NO: 106);
- Lipoprotein gi|256055301 or gi|365823350 or any *Borrelia* species homolog thereof is at least one peptide selected in the group comprising:
EISLTPEEAEKLESLK (SEQ ID NO: 107),
SLTEIYSGNGIPLVVSDVVK (SEQ ID NO: 108),
SLTEIDSGNGIPLVVSDVVK (SEQ ID NO: 109),
EFFDWLSK (SEQ ID NO: 110),
GEALSLFFQK (SEQ ID NO: 111) and
VLTESESNNELK (SEQ ID NO: 112);
- Hypothetical protein BbuN40_0563 or any *Borrelia* species homolog thereof is at least one peptide selected in the group comprising:
LPLALNIAVFR (SEQ ID NO: 113) and
LPLALNLAVSR (SEQ ID NO: 114);
- DbpA is at least one peptide selected in the group comprising:
TVTDAAEQHPTTTAEGILEIAK (SEQ ID NO: 115),
VSENSFILEAK (SEQ ID NO: 116),
DITDEIDAIK (SEQ ID NO: 117),
GVNFDAFK (SEQ ID NO: 118) and
TTANGIIEIVK (SEQ ID NO: 119).

14. Use of a set of polyeptides as defined in anyone of claims 11 to 14, in an ex *vivo* method of detecting *Borrelia burgdorferi* sensu lato in a subject likely to present an active Lyme infection.


SEQUENCE LISTING

<110>  UNIVERSITE DE STRASBOURG
       Centre National de la Recherche Scientifique

<120>  DETECTION OF BORRELIA BURGDORFERI SENSU LATO IN PATIENTS WITH
       LATE DISSEMINATED LYME INFECTION

<130>  BNT222087EP00

<160>  119

<170>  PatentIn version 3.5

<210>  1
<211>  13
<212>  PRT
<213>  Borrelia burgdorferi

<400>  1

Gly Ile Val Asp Ala Ala Gly Thr Ala Ala Gly Lys Lys
1               5                   10


<210>  2
<211>  12
<212>  PRT
<213>  Borrelia burgdorferi

<400>  2

Gly Ile Val Asp Ala Ala Gly Thr Ala Ala Gly Lys
1               5                   10


<210>  3
<211>  10
<212>  PRT
<213>  Borrelia burgdorferi

<400>  3

Asp Lys Val Ala Ala Ala Leu Val Leu Arg
1               5                   10


<210>  4
<211>  8
<212>  PRT
<213>  Borrelia burgdorferi

<400>  4

Val Ala Ala Ala Leu Val Leu Arg
1               5


<210>  5
<211>  15
<212>  PRT
<213>  Borrelia burgdorferi

<400>  5

Ala Ala Ala Ala Val Ser Ser Val Ser Gly Glu Gln Ile Leu Lys
1               5                   10                  15


<210>  6
<211>  12
<212>  PRT
<213>  Borrelia burgdorferi

<400>  6

Ala Val Ser Ser Val Ser Gly Glu Gln Ile Leu Lys
1               5                   10


<210>  7
<211>  12
<212>  PRT
<213>  Borrelia burgdorferi

<400>  7

Phe Ser Val Thr Asn Ala Asn Asp Ala Asn Val Lys
1               5                   10


<210>  8
<211>  17
<212>  PRT
<213>  Borrelia burgdorferi

<400>  8

Ile Gly Glu Ser Ala Asp Asn Gly Ala Ala Ala Asp Ala Asp Ser Val
1               5                   10                  15


Lys




<210>  9
<211>  15
<212>  PRT
<213>  Borrelia burgdorferi

<400>  9

Ala Ala Gly Ala Val Ser Ala Val Ser Gly Glu Gln Ile Leu Lys
1               5                   10                  15


<210>  10
<211>  12
<212>  PRT
<213>  Borrelia burgdorferi

<400>  10

Phe Ser Ala Ala Asp Gly Asp Gly Ala Asn Val Lys
1               5                   10

<210>  11
<211>  15
<212>  PRT
<213>  Borrelia burgdorferi

<400>  11

Ala Ala Glu Ala Val Ser Ser Val Ser Gly Glu Gln Ile Leu Lys
1               5                   10                  15


<210>  12
<211>  15
<212>  PRT
<213>  Borrelia burgdorferi

<400>  12

Ala Ala Glu Glu Ala Ile Val Gly Ala Thr Gly Asp Gly Thr Lys
1               5                   10                  15


<210>  13
<211>  12
<212>  PRT
<213>  Borrelia burgdorferi

<400>  13

Lys Asp Asp Gln Ile Ala Ala Ala Ile Ala Leu Arg
1               5                   10


<210>  14
<211>  11
<212>  PRT
<213>  Borrelia burgdorferi

<400>  14

Asp Asp Gln Ile Ala Ala Ala Ile Ala Leu Arg
1               5                   10


<210>  15
<211>  8
<212>  PRT
<213>  Borrelia burgdorferi

<400>  15

Ala Ile Val Asp Ala Ala Gly Lys
1               5


<210>  16
<211>  21
<212>  PRT
<213>  Borrelia burgdorferi

<400>  16

Asn Pro Ile Ala Ala Ala Ile Gly Asn Gly Asn Gly Ala Gly Phe Val
1               5                   10                  15

Asp Ala Asp Met Arg
            20

<210>  17
<211>  11
<212>  PRT
<213>  Borrelia burgdorferi

<400>  17

Asn Asn Gly Ile Asn Ala Ala Asn Leu Ser Lys
1               5                   10

<210>  18
<211>  11
<212>  PRT
<213>  Borrelia burgdorferi

<400>  18

Asn Asn Ala Ile Asn Ala Ala Asn Leu Ser Lys
1               5                   10

<210>  19
<211>  17
<212>  PRT
<213>  Borrelia burgdorferi

<400>  19

Ala Ile Asn Phe Ile Gln Thr Thr Glu Gly Asn Leu Asn Glu Val Glu
1               5                   10                  15

Lys

<210>  20
<211>  6
<212>  PRT
<213>  Borrelia burgdorferi

<400>  20

Ala Ser Tyr Ala Gln Lys
1               5

<210>  21
<211>  6
<212>  PRT
<213>  Borrelia burgdorferi

<400>  21

Ile Asn Ala Gln Ile Arg
1               5

<210>  22
<211>  11
<212>  PRT
<213>  Borrelia burgdorferi

<400>  22

Asp Ser Thr Glu Tyr Ala Ile Glu Asn Leu Lys
1               5                   10

<210>  23
<211>  16
<212>  PRT
<213>  Borrelia burgdorferi

<400>  23

Leu Glu Ser Ile Lys Asp Ser Thr Glu Tyr Ala Ile Glu Asn Leu Lys
1               5                   10                  15

<210>  24
<211>  13
<212>  PRT
<213>  Borrelia burgdorferi

<400>  24

Ala Ser Asp Asp Ala Ala Gly Met Gly Val Ser Gly Lys
1               5                   10

<210>  25
<211>  11
<212>  PRT
<213>  Borrelia burgdorferi

<400>  25

Asn Ser Thr Glu Tyr Ala Ile Glu Asn Leu Lys
1               5                   10

<210>  26
<211>  11
<212>  PRT
<213>  Borrelia burgdorferi

<400>  26

Thr Ala Glu Glu Leu Gly Met Gln Pro Ala Lys
1               5                   10

<210>  27
<211>  16
<212>  PRT

<213>  Borrelia burgdorferi

<400>  27

Ile Ala Asp Gln Ala Gln Tyr Asn Gln Met His Met Leu Ser Asn Lys
1               5                   10                  15


<210>  28
<211>  9
<212>  PRT
<213>  Borrelia burgdorferi

<400>  28

Ala Asn Leu Gly Ala Phe Gln Asn Arg
1               5


<210>  29
<211>  18
<212>  PRT
<213>  Borrelia burgdorferi

<400>  29

Met Lys Glu Leu Ala Val Gln Ser Gly Asn Gly Thr Tyr Ser Asp Ser
1               5                   10                  15


Asp Arg


<210>  30
<211>  16
<212>  PRT
<213>  Borrelia burgdorferi

<400>  30

Glu Leu Ala Val Gln Ser Gly Asn Gly Thr Tyr Ser Asp Ser Asp Arg
1               5                   10                  15


<210>  31
<211>  17
<212>  PRT
<213>  Borrelia burgdorferi

<400>  31

Ile Asn Thr Pro Ala Ser Leu Ser Gly Ser Gln Ala Ser Trp Thr Leu
1               5                   10                  15


Arg


<210>  32
<211>  14
<212>  PRT

<213>  Borrelia burgdorferi

<400>  32

Met Ile Ile Asn His Asn Thr Ser Ala Ile Asn Ala Ser Arg
1               5                   10


<210>  33
<211>  16
<212>  PRT
<213>  Borrelia burgdorferi

<400>  33

Gly Ser Ile Gln Ile Glu Ile Glu Gln Leu Thr Asp Glu Ile Asn Arg
1               5                   10                  15


<210>  34
<211>  8
<212>  PRT
<213>  Borrelia burgdorferi

<400>  34

Ile Ile Ala Glu Arg Asp Pro Lys
1               5


<210>  35
<211>  10
<212>  PRT
<213>  Borrelia burgdorferi

<400>  35

Ala Val Gly Leu Val Leu Pro Glu Leu Lys
1               5                   10


<210>  36
<211>  16
<212>  PRT
<213>  Borrelia burgdorferi

<400>  36

Val Pro Val Pro Thr Gly Ser Ile Val Asp Leu Thr Val Gln Leu Lys
1               5                   10                  15


<210>  37
<211>  14
<212>  PRT
<213>  Borrelia burgdorferi

<400>  37

Gly Ile Asp Ile Val Ala Ile Asn Asp Leu Thr Asp Pro Lys
1               5                   10

<210>  38
<211>  13
<212>  PRT
<213>  Borrelia burgdorferi

<400>  38

Gly Gly Tyr Leu Asp His Val Asn His Ala Gly Ala Lys
1               5                   10

<210>  39
<211>  12
<212>  PRT
<213>  Borrelia burgdorferi

<400>  39

Val Ile Leu Thr Val Pro Ala Lys Asp Glu Ile Lys
1               5                   10

<210>  40
<211>  16
<212>  PRT
<213>  Borrelia burgdorferi

<400>  40

Thr Ile Val Leu Gly Val Asn Asp His Asp Ile Asn Ser Asp Leu Lys
1               5                   10                  15

<210>  41
<211>  15
<212>  PRT
<213>  Borrelia burgdorferi

<400>  41

Ala Ala Ala Leu Ser Ile Ile Pro Thr Ser Thr Gly Ala Ala Lys
1               5                   10                  15

<210>  42
<211>  17
<212>  PRT
<213>  Borrelia burgdorferi

<400>  42

Ala Val Ser Asn Ala Ser Cys Thr Thr Asn Cys Leu Ala Pro Leu Ala
1               5                   10                  15

Lys

<210>  43
<211>  11
<212>  PRT
<213>  Borrelia burgdorferi

<400>  43

Ile Leu Asp Leu Pro His Ser Asp Leu Arg Arg
1               5                   10

<210>  44
<211>  10
<212>  PRT
<213>  Borrelia burgdorferi

<400>  44

Ile Leu Asp Leu Pro His Ser Asp Leu Arg
1               5                   10

<210>  45
<211>  24
<212>  PRT
<213>  Borrelia burgdorferi

<400>  45

Val Leu His Glu Ser Phe Gly Ile Glu Gln Gly Leu Met Thr Thr Val
1               5                   10                  15

His Ala Tyr Thr Asn Asp Gln Arg
            20

<210>  46
<211>  14
<212>  PRT
<213>  Borrelia burgdorferi

<400>  46

Val Leu Ser Trp Tyr Asp Asn Glu Phe Gly Tyr Ser Thr Arg
1               5                   10

<210>  47
<211>  8
<212>  PRT
<213>  Borrelia burgdorferi

<400>  47

Leu Ala Ile Asn Gly Phe Gly Arg
1               5

<210>  48
<211>  22
<212>  PRT
<213>  Borrelia burgdorferi

<400>  48

Asp Cys Val Asn Ala Asp Ile Val Val Ile Thr Ala Gly Leu Asn Gln

1               5                   10                  15

Lys Pro Gly Glu Thr Arg
            20

<210>  49
<211>  12
<212>  PRT
<213>  Borrelia burgdorferi

<400>  49

Gly Ala Thr Tyr Tyr Ala Ile Gly Leu Gly Ile Lys
1               5                   10

<210>  50
<211>  12
<212>  PRT
<213>  Borrelia burgdorferi

<400>  50

Ile Thr Glu Ile Glu Leu Asp Glu Ile His Lys Lys
1               5                   10

<210>  51
<211>  12
<212>  PRT
<213>  Borrelia burgdorferi

<400>  51

Val Ile Gly Thr Gly Thr Ile Leu Asp Thr Ser Arg
1               5                   10

<210>  52
<211>  14
<212>  PRT
<213>  Borrelia burgdorferi

<400>  52

Ile Ala Met Lys Pro Leu Ser Glu Tyr Leu Ser Glu Gly Lys
1               5                   10

<210>  53
<211>  29
<212>  PRT
<213>  Borrelia burgdorferi

<400>  53

Asn Ile Val Asn Ala Ile Ile Gly Asp Gln Asn Ile Ile Leu Pro Ile
1               5                   10                  15

Ser Ser Tyr Ile Asn Gly Gln Tyr Gly Gly Leu Ile Lys

20                  25

<210>  54
<211>  15
<212>  PRT
<213>  Borrelia burgdorferi

<400>  54

Ile Ile Lys Asp Asp Val Pro Asn Tyr Pro Leu Ala Ser Ser Lys
1               5                   10                  15

<210>  55
<211>  14
<212>  PRT
<213>  Borrelia burgdorferi

<400>  55

Ile Ser Met Pro Glu Gly Ser Phe Gln Asn Phe Ile Glu Lys
1               5                   10

<210>  56
<211>  10
<212>  PRT
<213>  Borrelia burgdorferi

<400>  56

Tyr Ala Gly Asp Thr Ile Leu Gly Val Arg
1               5                   10

<210>  57
<211>  11
<212>  PRT
<213>  Borrelia burgdorferi

<400>  57

Val Tyr Glu Thr Ser Gly Ala Glu Ser Leu Arg
1               5                   10

<210>  58
<211>  10
<212>  PRT
<213>  Borrelia burgdorferi

<400>  58

Asn Ser Val Val Ala Pro Ala Leu Val Lys
1               5                   10

<210>  59
<211>  15
<212>  PRT
<213>  Borrelia burgdorferi

<400>  59

Ala Glu Pro Gly Glu Leu Val Leu Asp Phe Ala Glu Leu Ala Arg
1               5                   10                  15


<210>  60
<211>  16
<212>  PRT
<213>  Borrelia burgdorferi

<400>  60

Arg Ala Glu Pro Gly Glu Leu Val Leu Asp Phe Ala Glu Leu Ala Arg
1               5                   10                  15


<210>  61
<211>  17
<212>  PRT
<213>  Borrelia burgdorferi

<400>  61

Gly Tyr Ala Thr Ser Val Gly Asp Glu Gly Gly Phe Ala Pro Asn Leu
1               5                   10                  15


Lys



<210>  62
<211>  18
<212>  PRT
<213>  Borrelia burgdorferi

<400>  62

Ser Ala Val Pro Ser Gly Ala Ser Thr Gly Ile Asn Glu Ala Val Glu
1               5                   10                  15


Leu Arg



<210>  63
<211>  18
<212>  PRT
<213>  Borrelia burgdorferi

<400>  63

Val Asn Gln Ile Gly Thr Leu Thr Glu Thr Phe Glu Ala Val Glu Met
1               5                   10                  15


Ala Lys



<210>  64

<211>  22
<212>  PRT
<213>  Borrelia burgdorferi

<400>  64

Ser Gly Glu Thr Glu Asp Thr Thr Ile Ala Asp Leu Val Val Ala Leu
1               5                   10                  15

Gly Thr Gly Gln Ile Lys
            20

<210>  65
<211>  9
<212>  PRT
<213>  Borrelia burgdorferi

<400>  65

Val Tyr Gln Tyr Leu Gly Ala Tyr Lys
1               5

<210>  66
<211>  17
<212>  PRT
<213>  Borrelia burgdorferi

<400>  66

Ile Gln Leu Val Gly Asp Asp Leu Phe Val Thr Asn Thr Ser Phe Leu
1               5                   10                  15

Lys

<210>  67
<211>  7
<212>  PRT
<213>  Borrelia burgdorferi

<400>  67

Ile Thr Ala Asp Ala Ile Arg
1               5

<210>  68
<211>  8
<212>  PRT
<213>  Borrelia burgdorferi

<400>  68

Ala Leu Thr Leu Ala Ile Asp Arg
1               5

<210>  69

<211>  17
<212>  PRT
<213>  Borrelia burgdorferi

<400>  69

Asp Phe Pro Ile Ala Pro Ile Tyr Ile Tyr Gly Asn Ser Tyr Leu Phe
1               5                   10                  15

Arg

<210>  70
<211>  9
<212>  PRT
<213>  Borrelia burgdorferi

<400>  70

Lys Ala Glu Glu Ile Ile Ile Glu Lys
1               5

<210>  71
<211>  7
<212>  PRT
<213>  Borrelia burgdorferi

<400>  71

Phe Asp Leu Ser Gln Leu Lys
1               5

<210>  72
<211>  18
<212>  PRT
<213>  Borrelia burgdorferi

<400>  72

Tyr Gly Gln Ser Trp Thr Asn Pro Glu Asn Ile Val Thr Ser Gly Pro
1               5                   10                  15

Phe Lys

<210>  73
<211>  27
<212>  PRT
<213>  Borrelia burgdorferi

<400>  73

Ser Asp Tyr Tyr Ser Ser Ala Val Asn Ala Ile Tyr Phe Tyr Ala Phe
1               5                   10                  15

Asn Thr His Ile Lys Pro Leu Asp Asn Val Lys

20                  25


<210>  74
<211>  11
<212>  PRT
<213>  Borrelia burgdorferi

<400>  74

Asn Leu Glu Leu Phe Asn Pro Glu Ile Ala Lys
1               5                   10


<210>  75
<211>  32
<212>  PRT
<213>  Borrelia burgdorferi

<400>  75

Thr Leu Glu Ile Thr Leu Glu Ser Pro Lys Pro Tyr Phe Ile Asp Met
1               5                   10                  15


Leu Val His Gln Ser Phe Ile Pro Ile Pro Ile His Ile Ala Glu Lys
            20                  25                  30


<210>  76
<211>  18
<212>  PRT
<213>  Borrelia burgdorferi

<400>  76

Gly Leu Ile Thr Gly Asp Pro Asn Thr Gly Gly Asn Lys Pro Gly Leu
1               5                   10                  15


Ala Lys



<210>  77
<211>  11
<212>  PRT
<213>  Borrelia burgdorferi

<400>  77

Glu Thr Gly Ser Asn Tyr Ser Glu Met Val Lys
1               5                   10


<210>  78
<211>  16
<212>  PRT
<213>  Borrelia burgdorferi

<400>  78

Ser Trp Asp Ile Ser Pro Asp Gly Thr Val Tyr Thr Phe Thr Leu Arg

1               5                   10                  15


<210>  79
<211>  8
<212>  PRT
<213>  Borrelia burgdorferi

<400>  79

Ile Ile Asp Ile Ile Ala Glu Arg
1               5


<210>  80
<211>  10
<212>  PRT
<213>  Borrelia burgdorferi

<400>  80

Lys Asp Asp Leu Glu Glu Ile His Ser Lys
1               5                   10


<210>  81
<211>  10
<212>  PRT
<213>  Borrelia burgdorferi

<400>  81

Thr Gln Asp Leu Tyr Glu Tyr Ile Glu Lys
1               5                   10


<210>  82
<211>  23
<212>  PRT
<213>  Borrelia burgdorferi

<400>  82

Leu Ile Asp Thr Ala Cys Asp Tyr Gly Thr Ala Asn Leu Ile Asp Asp
1               5                   10                  15


Ile Met Ser Asp Leu Glu Lys
            20


<210>  83
<211>  16
<212>  PRT
<213>  Borrelia burgdorferi

<400>  83

Leu Gln Glu Leu Leu Ala Ser Leu His Ile Phe Tyr Ser Asn Leu Arg
1               5                   10                  15


<210>  84

<211>  10
<212>  PRT
<213>  Borrelia burgdorferi

<400>  84

Ala Asn Asp Thr Leu Asn Ile Ile Leu Lys
1               5                   10


<210>  85
<211>  15
<212>  PRT
<213>  Borrelia burgdorferi

<400>  85

Leu Asn Ile Pro Thr Gly Ile Pro Leu Val Tyr Glu Leu Asp Lys
1               5                   10                  15


<210>  86
<211>  13
<212>  PRT
<213>  Borrelia burgdorferi

<400>  86

Gly Val Asp Glu Ala Ile Glu Ala Gly Leu Leu Leu Lys
1               5                   10


<210>  87
<211>  10
<212>  PRT
<213>  Borrelia burgdorferi

<400>  87

His Tyr Gly Ala Leu Gln Gly Leu Asn Lys
1               5                   10


<210>  88
<211>  21
<212>  PRT
<213>  Borrelia burgdorferi

<400>  88

Leu Asn Ile Pro Thr Gly Ile Pro Leu Val Tyr Glu Leu Asp Lys Asp
1               5                   10                  15


Leu Asn Pro Ile Lys
            20


<210>  89
<211>  19
<212>  PRT
<213>  Borrelia burgdorferi

<400>  89

Asp Ile Asp Lys Pro Phe Leu Leu Ala Val Glu Asp Val Phe Ser Ile
1               5                   10                  15

Ser Gly Arg

<210>  90
<211>  13
<212>  PRT
<213>  Borrelia burgdorferi

<400>  90

Thr Thr Asp Val Thr Gly Val Val Ala Leu Glu Gly Lys
1               5                   10

<210>  91
<211>  17
<212>  PRT
<213>  Borrelia burgdorferi

<400>  91

Gly Ser Ala Phe Gly Ala Met Ser Asn Pro Glu Asp Pro Glu Ala Thr
1               5                   10                  15

Lys

<210>  92
<211>  11
<212>  PRT
<213>  Borrelia burgdorferi

<400>  92

Tyr Gly Phe Ser Ala Asn Thr Pro Ile Ile Lys
1               5                   10

<210>  93
<211>  11
<212>  PRT
<213>  Borrelia burgdorferi

<400>  93

Val Gly Gln Glu Val Glu Ile Val Gly Ile Lys
1               5                   10

<210>  94
<211>  15
<212>  PRT
<213>  Borrelia burgdorferi

<400>  94

Gly Gln Val Leu Ser Ala Pro Gly Thr Ile Thr Pro His Lys Lys
1               5                   10                  15


<210>  95
<211>  14
<212>  PRT
<213>  Borrelia burgdorferi

<400>  95

Ala Leu Lys Tyr Glu Asp Ile Asp Asn Ala Pro Glu Glu Lys
1               5                   10


<210>  96
<211>  13
<212>  PRT
<213>  Borrelia burgdorferi

<400>  96

Thr Thr Leu Thr Ala Ala Ile Ser Ile Tyr Cys Ser Lys
1               5                   10


<210>  97
<211>  16
<212>  PRT
<213>  Borrelia burgdorferi

<400>  97

Ile Leu Glu Gln Gly Gln Ala Gly Asp Asn Val Gly Leu Leu Leu Arg
1               5                   10                  15


<210>  98
<211>  11
<212>  PRT
<213>  Borrelia burgdorferi

<400>  98

Tyr Glu Asp Ile Asp Asn Ala Pro Glu Glu Lys
1               5                   10


<210>  99
<211>  9
<212>  PRT
<213>  Borrelia burgdorferi

<400>  99

His Ile Glu Tyr Glu Thr Ala Asn Arg
1               5


<210>  100
<211>  8

<212>   PRT
<213>   Borrelia burgdorferi

<400>   100

Glu Leu Leu Pro Val Leu Glu Lys
1               5


<210>   101
<211>   27
<212>   PRT
<213>   Borrelia burgdorferi

<400>   101

Ser Ala Leu Gln Asn Ala Ala Ser Ile Ala Gly Leu Leu Leu Thr Thr
1               5                   10                  15


Glu Cys Ala Ile Thr Asp Ile Lys Glu Glu Lys
            20                  25


<210>   102
<211>   18
<212>   PRT
<213>   Borrelia burgdorferi

<400>   102

Gln Ile Ile Ser Asn Ala Gly Phe Glu Gly Ser Ile Tyr Ile His Gln
1               5                   10                  15


Ile Lys


<210>   103
<211>   29
<212>   PRT
<213>   Borrelia burgdorferi

<400>   103

Ala Ala Val Glu Glu Gly Val Val Pro Gly Gly Gly Ser Thr Leu Ile
1               5                   10                  15


Glu Val Ala Met Tyr Leu Asp Thr Ile Asp Thr Ser Lys
            20                  25


<210>   104
<211>   14
<212>   PRT
<213>   Borrelia burgdorferi

<400>   104

Val Asp Lys Asp Asn Thr Thr Ile Ile Asn Thr Gly Asn Lys
1               5                   10

<210>  105
<211>  12
<212>  PRT
<213>  Borrelia burgdorferi

<400>  105

Asn Val Ser Ser Gly Ile Asn Pro Ile Gly Ile Lys
1               5                   10


<210>  106
<211>  14
<212>  PRT
<213>  Borrelia burgdorferi

<400>  106

Trp Val Asn Met Ile Glu Ser Gly Ile Ile Asp Pro Ala Lys
1               5                   10


<210>  107
<211>  16
<212>  PRT
<213>  Borrelia burgdorferi

<400>  107

Glu Ile Ser Leu Thr Pro Glu Glu Ala Glu Lys Leu Glu Ser Leu Lys
1               5                   10                  15


<210>  108
<211>  20
<212>  PRT
<213>  Borrelia burgdorferi

<400>  108

Ser Leu Thr Glu Ile Tyr Ser Gly Asn Gly Ile Pro Leu Val Val Ser
1               5                   10                  15


Asp Val Val Lys
            20


<210>  109
<211>  20
<212>  PRT
<213>  Borrelia burgdorferi

<400>  109

Ser Leu Thr Glu Ile Asp Ser Gly Asn Gly Ile Pro Leu Val Val Ser
1               5                   10                  15


Asp Val Val Lys
            20

<210>  110
<211>  8
<212>  PRT
<213>  Borrelia burgdorferi

<400>  110

Glu Phe Phe Asp Trp Leu Ser Lys
1               5


<210>  111
<211>  10
<212>  PRT
<213>  Borrelia burgdorferi

<400>  111

Gly Glu Ala Leu Ser Leu Phe Phe Gln Lys
1               5                   10


<210>  112
<211>  12
<212>  PRT
<213>  Borrelia burgdorferi

<400>  112

Val Leu Thr Glu Ser Glu Ser Asn Asn Glu Leu Lys
1               5                   10


<210>  113
<211>  11
<212>  PRT
<213>  Borrelia burgdorferi

<400>  113

Leu Pro Leu Ala Leu Asn Ile Ala Val Phe Arg
1               5                   10


<210>  114
<211>  11
<212>  PRT
<213>  Borrelia burgdorferi

<400>  114

Leu Pro Leu Ala Leu Asn Leu Ala Val Ser Arg
1               5                   10


<210>  115
<211>  22
<212>  PRT
<213>  Borrelia burgdorferi

<400>  115

Thr Val Thr Asp Ala Ala Glu Gln His Pro Thr Thr Thr Ala Glu Gly
1               5                   10                  15

Ile Leu Glu Ile Ala Lys
            20

<210>  116
<211>  11
<212>  PRT
<213>  Borrelia burgdorferi

<400>  116

Val Ser Glu Asn Ser Phe Ile Leu Glu Ala Lys
1               5                   10

<210>  117
<211>  10
<212>  PRT
<213>  Borrelia burgdorferi

<400>  117

Asp Ile Thr Asp Glu Ile Asp Ala Ile Lys
1               5                   10

<210>  118
<211>  8
<212>  PRT
<213>  Borrelia burgdorferi

<400>  118

Gly Val Asn Phe Asp Ala Phe Lys
1               5

<210>  119
<211>  11
<212>  PRT
<213>  Borrelia burgdorferi

<400>  119

Thr Thr Ala Asn Gly Ile Ile Glu Ile Val Lys
1               5                   10